(19) Europäisches Patentamt
European Patent Office
Office européen des brevets

(11) Veröffentlichungsnummer: **0 213 523**
**B1**

(12) **EUROPÄISCHE PATENTSCHRIFT**

(45) Veröffentlichungstag der Patentschrift: **12.12.90**

(51) Int. Cl.$^5$: **A 61 K 9/50,** A 61 K 47/00

(21) Anmeldenummer: **86111368.6**

(22) Anmeldetag: **18.08.86**

(54) **Pharmazeutische Zusammensetzungen mit acylierten Phospholipiden.**

(30) Priorität: **19.08.85 US 766625**

(43) Veröffentlichungstag der Anmeldung:
**11.03.87 Patentblatt 87/11**

(45) Bekanntmachung des Hinweises auf die Patenterteilung:
**12.12.90 Patentblatt 90/50**

(84) Benannte Vertragsstaaten:
**AT BE CH DE FR GB IT LI LU NL SE**

(56) Entgegenhaltungen:
**EP-A-0 095 591**

**CHEMICAL ABSTRACTS, Band 100, Nr. 11, 12. März 1984, Seiten 391-392, Zusammenfassung Nr. 83795v, Columbus, Ohio, US; S.C. KINSKY et al.: "An alternative procedure for the preparation of immunogenic liposomal model membranes", & J. IMMUNOL. METHODS 1983, 65(3), 295-306**

**J. Immunol. meth. 65(1983), 295-306**

(73) Patentinhaber: **THE BOARD OF REGENTS UNIVERSITY OF TEXAS SYSTEM**
**6723 Bertner Avenue**
**Houston Texas 77030 (US)**

(72) Erfinder: **Schroit, Alan J., Dr.**
**8011 Portal Drive**
**Houston Texas 77071 (US)**
Erfinder: **Nayar, Rajiv, Dr.**
**6160 Elm Nbr 2804**
**Houston Texas 77081 (US)**

(74) Vertreter: **Zumstein, Fritz, Dr. et al**
**Bräuhausstrasse 4**
**D-8000 München 2 (DE)**

**EP 0 213 523 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der vorliegenden Erfindung ist eine pharmazeutische Zusammensetzung in Form einer Liposomendispersion mit acylierten Phosphatidylethanolamin-Derivaten, Phosphatidylethanolamin und Verbindungen mit pharmakologischen Eigenschaften. Die vorliegende Erfindung betrifft ebenfalls Mischungen aus acylierten Phosphatidylethanolamin-Derivaten und Phosphatidylethanolamin und Verbindungen mit pharmakologischen Eigenschaften, Verfahren zur Herstellung der pharmazeutischen Zusammensetzungen sowie deren Verwendung.

Die erfindungsgemässen pharmazeutischen Zusammensetzungen werden in Form von Liposomen angewendet.

Pharmazeutische Anwendungssysteme auf Liposomenbasis sind in dem Uebersichtswerk von G. Gregoriadis, Liposome Technology, Vol. II, Incorporation of Drugs and, Proteins and Genetic Material, CRC Press 1984, beschreiben.

Besondere Vorteile haben Verabreichungssysteme auf Liposomenbasis zur Einschleusung von Stoffen in endocytierende Zellen, insbesondere des Retikuloendothelial-Systems. So beobachtet man beispielsweise eine Erleichterung des Transports von Antiobiotika in endocytierende Zellen und eine verbesserte Bekämpfung der in diesen Zellen befindlichen Erreger. Endocytierende Zellen sind auch bei entzündlichen Prozessen beteiligt. Man beobachtet eine schnellere Einschleusung von Liposomenverkapselten antirheumatischen Wirkstoffen in solche Zellen als in das umliegende Gewebe. In Form von Liposomen verkapselte Zytostatika können in die spezifischen Organe des Retikuloendothelial-Systems (Leber, Milz, Knochenmark) eingeschleust, oder es können in der Lunge infolge von Filtration in den Lungenkapillaren und anschliessendem Transport durch auswandernde Blutmonocyten Wirkstoffe in alveolären Makrophagen angereichert und damit eine Verbesserung der Wirkung auf Lungen- oder Lebertumore bei gleichzeitiger Verminderung der Toxizität erreicht werden.

Im J. of Immunological Methods 65 (1983) 295—306 sind Liposomendispersionen mit N-Succinylphosphatidylethanolaminderivaten beschrieben, worin die N-Succinylgruppe durch Schutzgruppen blockiert ist. Das N-Succinylphosphatidylethanolamin-Derivat wird in Liposomen an Haptene wie Dinitrophenyllysin gebunden, welche *in vivo* eine Immunostimulierung auslösen können.

Es wurde überraschenderweise gefunden, dass die Immunstimulierung wie Aufnahme von Liposomen und ihre Endocytose durch Makrophagen, insbesondere alveoläre Makrophagen, verbessert wird, wenn acylierte Phosphatidylethanolamin-Derivate mit freier Carboxygruppe, in die Schalenstruktur von Liposomen ohne Bindung an Haptene eingebaut werden.

Die vorliegende Erfindung betrifft pharmazeutische Zusammensetzungen in Form einer Liposomendispersion bestehend aus

a) einen Phospholipid der Formel

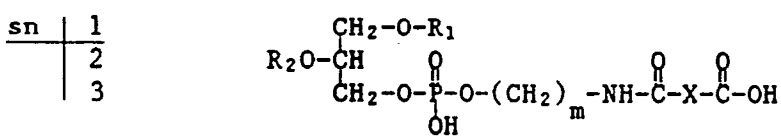

(I),

worin m zwei oder drei, $R_1$ und $R_2$ unabhängig voneinander Alkyl, Alkenyl oder Acyl mit je 10 bis 20 C-Atomen und X die direkte Bindung, $C_1$—$C_4$-Alkylen, $C_2$—$C_4$-Alkenylen oder $C_1$—$C_4$-Alkylen oder $C_2$—$C_4$-Alkenyl substituiert durch Hydroxy bedeuten, oder ein pharmazeutisch annehmbares Salz davon,

b) einem Phospholipid der Formel

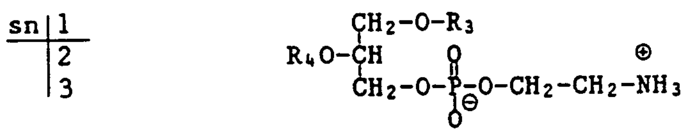

(II),

worin $R_3$ und $R_4$ die Acylgruppe einer gesättigten oder ungesättigten Carbonsäure mit 10—20 C-Atomen und 1—2 Doppelbindungen bedeuten.

c) einer Verbindung oder einer Mischung von Verbindungen mit pharmakologischen Eigenschaften und gegebenenfalls

d) einem Lipid aus der Gruppe Phosphatidylcholin, Phosphatidylserin, Phosphatidylinositol, Phosphatidylglycerin, Cardiolipin und Cholesterin und gegebenenfalls einer pharmazeutisch annehmbaren Trägerlösung, welche auf pH 7,0—7,8 gepuffert ist, und pharmazeutisch annehmbaren Hilfsstoffen.

Die weiter vorn und im folgenden genannten allgemeinen Begriffe haben im Rahmen der Beschreibung der vorliegenden Erfindung vorzugsweise die folgenden Bedeutungen:

Der im Zusammenhang mit organischen Resten, z.B. Niederalkyl, Niederalkylen, Niederalkoxy, Niederalkanoyl etc., verwendete Ausdruck "Nieder" bedeutet, dass solche organischen Reste, falls nicht ausdrücklich anders definiert, bis einschliesslich 7 und bevorzugt bis einschliesslich 4 Kohlenstoffatome enthalten.

Die Nomenklatur der Phospholipide der Formeln I und II erfolgt anhand der in Eur. J. of Biochem. 79, 11—21 (1977) "Nomenclature of Lipids" von der IUPAC—IUB Commission on Biochemical Nomenclature (CBN) gegeben Empfehlungen (sn-Nomenklatur, stereospecific numbering).

Für die pharmazeutischen Wirkstoffe werden, wenn nicht anders angegeben, die von der Weltgesundheitsorganisation (WHO) vorgeschlagenen Freinamen (Recommended International Nonproprietary Names) verwendet, welche dem Standardwerk "Pharmazeutische Chemie" (E. Schröder, C. Rufer und R. Schmiechen, Thieme Verlag Stuttgart, 1982), sowie dem Merck-Index (Tenth Edition), entnommen wurden.

In einem Phospholipid der Formel I (Komponente a)) ist m vorzugsweise zwei.

Alkyl $R_1$ und/oder $R_2$ ist vorzugsweise geradkettig mit einer geraden Anzahl von 10 bis 20 C-Atomen, z.B. n-Decyl, n-Dodecyl, n-Tetradecyl, n-Hexadecyl, n-Octadecyl oder n-Icosyl.

Alkenyl $R_1$ und/oder $R_2$ ist vorzugsweise geradkettige mit einer geraden Anzahl von 12 bis 20 C-Atomen und 1 Doppelbindung, z.B. 9-cis-Dodecenyl, 9-cis-Tetradecenyl, 9-cis-Hexadecenyl, 6-cis-Octadecenyl, 6-trans-Octadecenyl, 9-cis-Octadecenyl, 9-trans-Octadecenyl oder 9-cis-Icosenyl.

Acyl $R_1$ und/oder $R_2$ ist vorzugsweise geradkettig mit einer geraden Anzahl von 10—20 C-Atomen, z.B. $C_{10}$—$C_{20}$-Alkanoyl oder $C_{10}$—$C_{20}$-Alkenoyl.

Alkanoyl $R_1$ und/oder $R_2$ ist vorzugsweise n-Decanoyl, n-Dodecanoyl, n-Tetradecanoyl, n-Hexadecanoyl, n-Octadecanoyl und n-Icosanoyl.

Alkenoyl $R_1$ und/oder $R_2$ ist vorzugsweise 9-cis-Dodecenoyl, 9-cis-Tetradecenoyl, 9-cis-Hexadecenoyl, 6-cis-Octadecenoyl, 6-trans-Octadecenoyl, 9-cis-Octadecenoyl, 9-trans-Octadecenoyl, 11-cis-Octadecenoyl und 9-cis-Icosenoyl.

X mit der Bedeutung $C_1$—$C_4$-Alkylen ist geradkettiges oder verzweigtes $C_1$—$C_4$-Alkylen, z.B. Methylen, 1,1-Ethylen, 1,1-, 1,2- oder 1,3-Propylene oder vorzugsweise 1,2-Ethylen.

X mit der Bedeutung $C_2$—$C_4$-Alkenylen ist geradkettiges oder verzweigtes Alkenylen, z.B. Vinylen, Propenylen oder 1,2- oder 2,3-Butenylen.

X mit de Bedeutung $C_1$—$C_4$-Alkylen substituiert durch Hydroxy ist vorzugsweise geradkettiges $C_1$—$C_4$-Alkylen substituiert durch eine oder in Abhängigkeit von der Gesamtzahl an C-Atomen durch bis zu 4 Hydroxygruppen, z.B. 1-Hydroxy-1,2-ethylen, 1,2-Dihydroxy-1,2-ethylen, 1- oder 2-Hydroxy-1,3-propylen oder 1,2-Dihydroxy-1,3-propylen.

Ein pharmazeutisch annehmbares Salz des Phospholipids (I) wird vorzugsweise durch Reaktion mit einem oder zwei Aequivalenten verdünnter wässriger Alkalimetallhydroxid-Lösung gebildet, z.B. Natrium- oder Kaliumhydroxid-Lösung und ist vorzugsweise das Natrium- oder Dinatriumsalz.

Weitere pharmazeutisch annehmbare Salze werden durch Reaktion mit Aminen wie Trimethyl-, Ethyl-, Diethyl- oder Triethylamin, Piperidin, Piperazin, 2-Hydroxyethylpiperazin, Cyclohexylamin, Pyrrolidin, oder Cholin gebildet.

In einem Phospholipid (I) sind $R_1$ und $R_2$ vorzugsweise geradkettiges Alkenoyl mit einer geraden Anzahl von 10 bis 20 C-Atomen, z.B. 9-cis-Dodecenoyl, 9-cis-Tetradecenoyl, 9-cis-Hexadecenoyl, 6-cis-, 6-trans-, 9-cis-, 9-trans-, oder 11-cis-Octadecenoyl oder 9-cis-Icosenoyl, X ist $C_2$—$C_4$-Alkylen, z.B. 1,2-Aethylen oder 1,3-Propylen, oder $C_2$—$C_4$-Alkenylen, z.B. Vinylen.

Besonders bevorzugt sind die Natrium- und Dinatriumsalze des N-[1,2-Di-(9-cis-octadecenoyl)-sn-glycero-3-phosphoethanol)-N-hydroxysuccinylamins und N-[1,2-Di-(9-cis-octadecenoyl)-sn-glycero-3-phosphoethanol]-N-glutarylamins.

In einem Phospholipid (II) — Komponente b) — stellen die Acylgruppen $R_3$ und $R_4$ vorzugsweise geradkettiges $C_{10}$—$C_{20}$-Alkanoyl oder $C_{10}$—$C_{20}$-Alkenoyl mit gerader Anzahl an C-Atomen dar, z.B. 9-cis-Dodecenoyl, 9-cis-Tetradecenoyl, 9-cis-Hexadecenoyl oder 6-cis-, 6-trans-, 9-cis-, 9-trans- oder 11-cis-Octadecenoyl.

Besonders bevorzugt ist 1,2-Di-(9-cis-octadecenoyl)-sn-glycero-3-phosphoethanolamin.

Verbindungen oder Mischungen von Verbindungen mit pharmakologischen Eigenschaften sind in erster Linie pharmazeutische Wirkstoffe und Stoffgemische aus der Gruppe der Antiphlogistika, Antibiotika, Antileishmanien, Antimykotika, Antineoplastika und Immunmodulatoren.

Pharmazeutische Wirkstoffe aus der Gruppe der Antiphlogistica sind z.B. Glucocorticoide, z.B. Cortison, Hydrocortison, Prednison, Prednisolon, Fluocortolon, Triamcinolon, Methylprednisolon, Prednyliden, Paramethason, Dexamethason, Betamethason, Beclomethason, Fluprednyliden, Desoximethason, Fluocinolon, Flumethason, Diflucortolon, Clocortolon, Clobetasol oder Fluocortinbutylester, nicht-steroide Entzündungshemmer aus der Gruppe der substituierten Phenylessigsäuresalze oder 2-Phenylpropionsäuresalze, z.B. Alclofenac, Ibufenac, Ibuprofen, MK—830, BL—2365, Clindanac, Fenclorac, Ketoprofen, Fenoprofen, Indoprofen, Fenclofenac, Diclofenac, Flurbiprofen, Pirprofen, Naproxen, Benoxaprofen, Carprofen oder Cicloprofen, Anthranilsäure-Derivate, z.B. der Formel

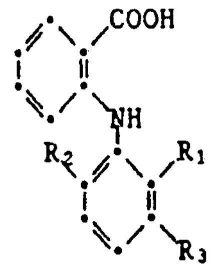

(III),

worin $R_1$, $R_2$ und $R_3$ unabhängig voneinander Wasserstoff, Methyl, Chlor oder Trifluormethyl bedeuten, z.B. Mefenaminsäure, Flufenaminsäure, Tolfenaminsäure oder Meclofenaminsäure, Anilino-substituierte Nicotinsäure-Derivate, z.B. Mifluminsäure, Clonixin oder Flunixin, Heteroarylessigsäuren oder 2-Heteroarylessigsäuren mit einem 2-Indol-3-yl- oder Pyrrol-2-yl-Rest, z.B. Indometacin, Oxmetacin, Intrazol, Acemetazin, Cinmetacin, Zomepirac, Tolmetin, Colpirac oder Tiaprofensäure, Indenylessigsäurederivate vom Sulindac-Typ, sowie analgetisch wirksame Heteroaryloxyessigsäuren, z.B. Benzadac.

Pharmazeutische Wirkstoffe aus der Gruppe der Antibiotika sind z.B. Tetracyclin-Antibiotika der Formel

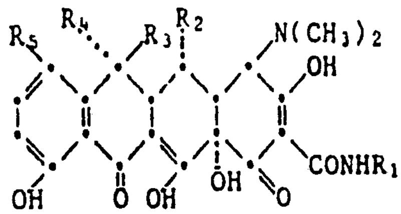

(V),

worin $R_1$ Wasserstoff oder Pyrrolidin-1-ylmethyl, $R_2$ Wasserstoff oder Hydroxy, $R_3$ Wasserstoff, Hydroxy oder Methyl, $R_4$ Wasserstoff oder Methyl und $R_5$ Wasserstoff, Chlor oder Dimethylamino bedeuten, z.B. Chlortetracyclin, Oxytetracyclin, Tetracyclin, Demethylchlortetracyclin, Metacyclin, Doxycyclin, Minocyclin oder Rolitetracyclin, Aminoglycoside, z.B. Kanamycin, Amikacin, Gentamycin $C_{1a}$, $C_2$, $C_{2b}$ oder $C_1$, Sisomicin, Netilmicin, Spectinomycin, Streptomycin, Tobramycin, Neomycin B, Dibekacin oder Kanendomycin, Makrolide, z.B. Maridomycin oder Erythromycin, Lincomycine, z.B. Clindamycin oder Lincomycin, Penicillansäure (6-APA)- und Cephalosporansäure (7-ACA)- Derivate mit 6β- bzw. 7β-Acylaminogruppen, welche in fermentativ, halb- oder totalsynthetisch erhältlichen 6β-Acylaminopenicillansäure- oder 7β-Acylaminocephalosporansäurederivaten oder in 3-Stellung abgewandelten 7β-Acylaminocephalosporansäurederivaten vorhanden sind, z.B. unter den Namen Penicillin G oder V, Phenethicillin, Propicillin, Nafcillin, Oxacillin, Cloxazillin, Dicloxazillin, Flucloxacillin, Cyclazillin, Epicillin, Mecillinam, Methicillin, Azlocillin, Sulbenicillin, Ticarcillin, Mezlocillin, Piperacillin, Carindacillin, Azidocillin oder Ciclazillin bekannt gewordene Penicillansäurederivate oder unter den Namen Cefaclor, Cefuroxim, Cefazlur, Cephacetril, Cefazolin, Cephalexin, Cefadroxil, Cephaloglycin, Cefoxitin, Cephaloridin, Cefsulodin, Cefotiam, Ceftazidin, Cefonicid, Cefotaxim, Cefmenoxim, Ceftizoxim, Cephalothin, Cephradin, Cefamandol, Cephanon, Cephapirin, Cefroxadin, Cefatrizin, Cefazedon, Ceftrixon oder Ceforanid bekannt gewordene Cephalosporinderivate, sowie andere β-Lactam-Antibiotica vom Clavam-, Penem- oder Carbapenem-Typ, z.B. Moxalactam, Clavulansäure, Nocardicin A, Sulbactam, Aztreonam oder Thienamycin, sowie Antibiotika vom Bicozamycin-, Novobiocin-, Chlor- oder Thiamphenicol-, Rifampicin-, Fosfomycin-, Colistin- oder Vancomycin-Typ.

Pharmazeutische Wirkstoffe aus der Gruppe der Antileishmanien sind z.B. Antimon-Verbindungen, z.B. Brechweinstein (Kalium-antimonyl-tartrat), Stibophen, Natriumstibocaptat sowie Natriumstibogluconat.

Pharmazeutische Wirkstoffe aus der Gruppe der Antimykotika sind z.B. Thiokohlensäurederivate, z.B. Dibenzthion, Tolnaftat oder Tolciclat, Imidazol-Derivate, z.B. Clotrimazol, Miconazol, Econazol, Isoconazol oder Ketoconazol oder Polyen-Antibiotika, z.B. Nystatin, Natamycin oder Amphotericin B.

Pharmazeutische Wirkstoffe aus der Gruppe der Antineoplastika sind beispielsweise Alkylanzien mit Bis-(2-chloräthyl)-amin-Gruppe, z.B. Chlormethin, Chlorambucil, Melphalan, Uramustin, Mannomustin, Estramustinphosphat, Mechlorethaminoxid, Cyclophosphamid, Ifosfamid oder Trifosfamid, Alkylanzien mit Aziridin-Struktur, z.B. Tretamin, Thiotepa, Triaziquon oder Mitomycin, alkylierende Methansulfonsäureester, z.B. Busulfan, alkylierende N-Alkyl-N-nitrosoharnstoff-Derivate, z.B. Carmustin, Lomustin, Semustin oder Streptozotocin, sowie Alkylanzien vom Mitobronitol-, Dacarbazin- oder Procarbazin-Typ, Antimetabolite vom Folsäure-Typ, z.B. Methotrexat, Purin-Derivate, z.B. Mercaptopurin, Thioguanin, Azathioprin, Thiamiprin, Vidarabin oder Puromycin, Pyrimidin-Derivate, z.B. Fluorouracil, Floxuridin, Tegafur, Cytarabin, Idoxuridin, Flucytosin, Antibiotika, welche in der Krebschemotherapie eingesetzt werden, z.B. Dactinomycin, Daunorubicin, Doxorubicin, Mithramycin, Bleomycin $A_2$ oder $B_2$ oder Etoposid, sowie Vinca-Alkaloide, z.B. Vincristin, gegebenenfalls in Kombination mit Chlormethamin, Prednisolon oder Prednison und Procarbazin.

Immunmodulatoren sind z.B. Muramylpeptide, z.B. Muramyldipeptide oder Muramyltripeptide, insbesondere der Formel

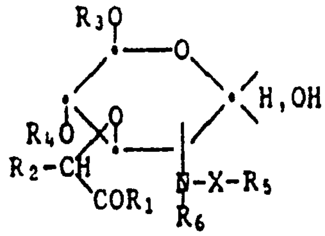

(VI),

worin X die Gruppen —C(=O)— oder —C(=O)—O—, $R_1$ die L - Ala - D - isoGln - L - Ala - 2 - (1,2 - dipalmitoyl - sn - glycero - 3 - hydroxyphosphoryloxy) - ethylamid -, L - Ala - D - Glu(Cy - L - Ala - 2 - (1,2 - dipalmitoyl - sn - glycero - 3 - hydroxyphosphoryloxy) - ethylamid -, L - Ala - D - isoGlnOH -, L - Ala - D - GlnNH$_2$ - α - n - butylester -, L - Ala - D - isoGln - L - (stearoyl) - Lys -, L - Val - D - Gln - NH$_2$ - α - n - methylester -, L - Ala - D - isoGln - L - Ala - 1,2 - dipalmitoyl - sn - glycerinester- oder die L - Ala - D - isoGln - L - Ala - cholesterinester-Gruppe, $R_2$ Wasserstoff, Methyl oder n-Propyl, $R_3$ Wasserstoff, n-Stearoyl, 10-(2,3-Dimethoxy-1,4-dioxo-5-methyl)-2,5-cyclohexadienoyl, 2-Behenoyloxy-2-methylpropanoyl oder n-Octanoyl, $R_4$ Wasserstoff oder n-Octanoyl bedeuten, $R_5$ $C_1$—$C_4$-Alkyl und $R_6$ Wasserstoff oder $C_1$—$C_4$-Alkyl sowie die entsprechenden 2-Palmitoylthio-Derivate davon, Lipopeptide mit immunmodulatorischen Eigenschaften vom Typ n - Lauroyl - L - Ala - D - isoGln - (m - DAP - Gly) - NH$_2$, n - Lauroyl - L - Ala - D - isoGln - (L - DAP - Gly) - NH$_2$, n - Lauroyl - L - Ala - D - isoGln - (L - Lys - D - Ala) - NH$_2$, n - Octanoyl - L - Ala - D - isoGln(L - Lys - D - Ala) - NH$_2$ oder Palmitoyl - Cys - ((2R) - 2,3 - dilauroyloxypropyl) - Ala - D - Glu - (Gly - taurin - Na) - NH$_2$, oder sind Lymphokine, die von Lymphozyten, Monozyten oder Makrophagen ausgeschieden werden, wenn diese durch Antigene oder Mitogene oder dergleichen stimuliert werden.

Zur Gruppe der Lymphokine gehören beispielsweise bekannte Interferon-Typen, insbesondere natürliches oder rekombiniertes, humanes Gamma-Interferon, z.B. humanes Gamma-Interferon, das gemäss den Europäischen Patentanmeldungen 63,482, 77,670, 83,777, 88,540, 89,676, 95,350, 99,084, 110,044 und 112,967 und den Internationalen Anmeldungen (PCT) WO 83/04,053 und WO 84/02,129 erhältlich ist.

Bevorzugt ist rekombiniertes humanes Gamma-Interferon gemäss Europäischer Patentanmeldung 121,157 mit folgender Aminosäuresequenze:

H$_2$N-Cys-Tyr-Cys-Gln-Asp-Pro-Tyr-Val-Gln-Glu-Ala-Glu-Asn-Leu-Lys-Lys-
Tyr-Phe-Asn-Ala-Gly-His-Ser-Asp-Val-Ala-Asp-Asn-Gly-Thr-Leu-Phe-Leu-
Gly-Ile-Leu-Lys-Asn-Trp-Lys-Glu-Glu-Ser-Asp-Arg-Lys-Ile-Met-Gln-Ser-
Gln-Ile-Val-Ser-Phe-Tyr-Phe-Lys-Leu-Phe-Lys-Asn-Phe-Lys-Asp-Asp-Gln-
Ser-Ile-Gln-Lys-Ser-Val-Glu-Thr-Ile-Lys-Glu-Asp-Met-Asn-Val-Lys-Phe-
Phe-Asn-Ser-Asn-Lys-Lys-Lys-Arg-Asp-Asp-Phe-Glu-Lys-Leu-Thr-Asn-Tyr-
Ser-Val-Thr-Asp-Leu-Asn-Val-Gln-Arg-Lys-Ala-Ile-His-Glu-Leu-Ile-Gln-
Val-Met-Ala-Glu-Leu-Ser-Pro-Ala-Ala-Lys-Thr-Glu-Lys-Arg-Lys-Arg-Ser-
Gln-Met-Leu-Phe-Gln-Gly-Arg-Arg-Ala-Ser-Gln-OH,

und rekombiniertes humanes Gamme-Interferon gemäss Britischer Patentanmeldung 2,107,718 mit folgender Aminosäuresequenz:

H$_2$N-Cys-Tyr-Cys-Gln-Asp-Pro-Tyr-Val-Lys-Glu-Ala-
Glu-Asn-Leu-Lys-Lys-Tyr-Phe-Asn-Ala-Gly-His-Ser-
Asp-Val-Ala-Asp-Asn-Gly-Thr-Leu-Phe-Leu-Gly-Ile-
Leu-Lys-Asn-Trp-Lys-Glu-Glu-Ser-Asp-Arg-Lys-Ile-
Met-Gln-Ser-Gln-Ile-Val-Ser-Phe-Tyr-Phe-Lys-Leu-
Phe-Lys-Asn-Phe-Lys-Asp-Asp-Gln-Ser-Ile-Gln-Lys-
Ser-Val-Glu-Thr-Ile-Lys-Glu-Asp-Met-Asn-Val-Lys-
Phe-Phe-Asn-Ser-Asn-Lys-Lys-Lys-Arg-Asp-Asp-Phe-

Glu-Lys-Leu-Thr-Asn-Tyr-Ser-Val-Thr-Asp-Leu-Asn-
Val-Gln-Arg-Lys-Ala-Ile-His-Glu-Leu-Ile-Gln-Val-
Met-Ala-Glu-Leu-Ser-Pro-Ala-Ala-Lys-Thr-Gly-Lys-
Arg-Lys-Arg-Ser-Gln-Met-Leu-Phe-Arg-Gly-Arg-Arg-
Ala-Ser-Gln-OH.

Zur Gruppe der Lymphokine gehört ausserdem in gereinigter Form vorliegendes, humanes Interleukin 2, z.B. nach Aktivierung von humanen, neoplastischen Leukämie- oder Lymphom-Zellen durch T-Zellmitogen im Kulturfiltrat erhältliches und durch Umkehrphasen-HPLC-gereinigtes Interleukin 2, aus Kulturen mit menschlichen T-Lymphozyten aus Milz oder peripherem Blut nach Stimulierung durch Antigene oder Mitogene, z.B. humane T-Zellen-Leukämie-Lymphom-Viren (HTLV—I) Phytohämagglutinin oder Concanavalin A, erhältliche Kulturfiltrate, welche Gemische mit den unter den Begriffen Makrophagen-Migration-Inhibitionsfaktor (MIF), Leukozyten-Migration-Inhibitionsfaktor, Leukocyten-Migration-Verstärkungsfaktor, Makrophagen-Aktivierender-Faktor (MAF), Kolonien-stimulierender-Faktor, Interleukin 1 und 2 sowie Gamma-Interferon bekannt gewordenen Bestandteilen enthalten, insbesondere solche Kulturfiltrate oder Isolate mit einem hohen Anteil an Makrophagen-Aktivierendem-Faktor (MAF).

Bevorzugt sind N - Acetyl - D - muramyl - L - alanyl - D - isoglutaminyl - L - alanin - 2 - (1,2 - dipalmitoyl - sn - glycero - 3 - hydroxyphosphoryloxy) - ethylamid, Natrium - N - acetyl - D - muramyl - L - alanyl - D - isoglutamin oder Natrium - N - acetyldesmethylmuramyl - L - alanyl - D - isoglutamin der Formel V, gegebenenfalls in Kombination mit gereinigtem, natürlichem oder rekombiniertem humanem Gamma Interferon.

Lipide — Komponente d) — aus der Gruppe der Phosphatidylcholine, Phosphatidylserin, Phosphatidylinositol, Phosphatidylglycerin und Cardiolipin sind synthetische Phospholipide oder natürliche Phospholipide mit verschiedenen Acylgruppen von unterschiedlichem Molgewicht und unterschiedlicher Struktur, z.B. Phosphatidylcholin aus Sojabohnen oder Hühnerei oder Phosphatidylcholin aus dem Rinderhirn, Rinderleber oder Schweineleber, Phosphatidylserin aus dem Rinderhirn, Phosphatidylinositol aus Sojabohnen oder aus Hefe, Phosphatidylglycerin aus Eigelb oder Cardiolipin aus dem Schweineherz.

Cholesterinderivate sind zum Beispiel Cholestan, Coprostan, Ergosterol oder Stigmasterol.

Die pharmazeutischen Zusammensetzungen der vorliegenden Erfindung besitzen in Form von Liposomen angewandt ausgezeichnete phagocytische Eigenschaften. Beispielsweise ist die Phagocytose von multilamellaren Liposomen bestehend aus einer 3:7-Mischung von Natrium - N - [1,2 - di - (9 - cis - octadecenoyl) - sn - glycero - 3 - phospoethanol] - N - hydroxysuccinylamin (I) und Dioleoylphosphatidylethanolamin (II) oder Natrium - N - [1,2 - di - (9 - cis - octadecenoyl) - sn - glycero - 3 - phosphoethanol] - N - hydroxyglutarylamin (I) und Dioleoylphosphatidylethanolamin (II) durch peritoneale Makrophagen von Mäusen höher als die Phagocytose stimuliert durch multilamellare Liposomen, welche ausschliesslich aus einer 3:7 molaren Mischung aus Phosphatidylserin und Phosphatidylcholin bestehen. Dieser Befund ist *in-vitro* durch Inkubierung von peritonealen Makrophagen von Mäusen mit multilamellaren Liposomen, welche Spuren von radioaktivem $^{125}I$ als Markierung enthalten, nachweisbar. Bei der experimentellen Ausführung werden in regelmässigen Abständen die Kulturen gewaschen und die Menge an Strahlung, welche von markierten Zellen ausgeht, gemessen. Ebenso zeigen multilamellare Liposomen, welche aus einer 3:7-Mischung von Phospholipiden der Formeln I und II bestehen und Immunomodulatoren wie MDP oder Gamma-Interferon enthalten, verstärkte Makrophagenstimulanz bis hin zu einer tumoriciden Wirkung bei niedrigeren Dosen als multilamellare Liposomen, welche aus Phosphatidylcholin und Phosphatidylserin bestehen und die gleiche Menge MDP und Gamma-Interferon enthalten. Dieser Effekt ist ebenfalls *in-vitro* nachweisbar, indem man peritoneale Makrophagen von Mäusen in Kulturschalen plattiert und mit multilamellaren Liposomen in Lösung enthaltend Natrium - N - [1,2 - Di - (9 - cis - octadecenoyl) - sn - glycero - 3 - phosphoethanol] - N - hydroxysuccinylamin (I) und Dioleoylphosphatidylethanolamin (II) in einem molaren 3:7 Verhältnis und MDP und Gamma-Interferon als Wirkstoffe und mit multilamellaren Liposomen enthaltend Phosphatidylcholin und Phosphatidylserin im selben Molverhältnis und dieselbe Menge MDP und Gamma-Interferon aktiviert. Die Liposomen-Zubereitungen werden bei einer Konzentration von 100 nmol Phospholipid-Gesamtgehalt pro Schale enthaltend 6 Einheiten rekombiniertes Gamma-Interferon und 0,2 g MDP miteinander verglichen. Nach einem Waschvorgang werden $10^4$ [$^{125}$I]Iododeoxyuridinmarkierte BL6-Melanomazellen hinzugefügt. Die Zytotoxizität wird nach 72 Stunden Kultivierung durch Messung der Radioaktivität, welche von den adhärenten lebensfähigen Zielzellen ausgeht, bestimmt, indem man die Kulturen mindestens dreimal mit HBSS (Hank's Balanced Salt Solution) wäscht. Die prozentuale Zytotoxizität kann durch Bestimmung der radioaktiven Impulse pro Minute verglichen mit Kontrollkulturen enthaltend nicht-aktivierte Makrophagen und Zielzellen ermittelt werden.

Die pharmazeutischen Zusammensetzungen der vorliegenden Erfindung in Form von multilamellaren Liposomen zeichnen sich durch hervorragende Abgabeeigenschaften bei niedrigen pH-Werten aus. So werden die Abgabeeigenschaften von Liposomen bestehend aus Dinatrium - N - [1,2 - di - (9 - cis - octadecenoyl) - sn - glycero - 3 - phosphoethanol] - N - hydroxysuccinylamin und 1,2 - Di - (9 - cis - octadecenoyl) - sn - glycero - 3 - phosphoethanolamin in einem molaren 3:7-Verhältnis nach der Fluoreszenzmethode von Ellens et al. Biochemistry 1984, 23, 1532—1538 bestimmt, um die Abgabe von Verbindungen aus Liposomen bei niedrigen pH-Werten zu ermitteln. Zur Bestimmung der pH-Abgängigkeit der Abgabe werden Liposomen in Pufferlösungen mit pH-Werten von 4,0 bis 7,4 injiziert und der Prozentsatz von noch verkapseltem 8-Aminonaphthalin-1,3,6-trisulfonsäure (ANTS) und p-Xylylen-bispyridiniumdibromid (DPX), welche in dieser Versuchsanordnung die verkapselten Wirkstoffe ersetzen, ermittelt. Vollständige Verkapselung der wasserlöslichen fluoreszierenden ANTS, welche in einem Komplex mit dem "Quencher" DPX vorliegt, löscht die von ANTS ausgehende Fluoreszenz fast vollständig. Die Abgabe von ANTS aus den Liposomen kann durch Zunahme der Fluoreszenz verfolgt werden.

Es wurde gefunden, dass oberhalb von pH 6,0 kaum Abgabe erfolgt. Bei Abnahme des pH verstärkt sich die Abgabe von ANTS/DPX aus den Liposomen, wobei die halbe Abgabemenge bei pH 4,5 und die vollständige Abgabemenge bei pH 4,0 gemessen wird.

Es ist bekannt, dass pathologische Gewebe in ihrer umittelbaren Umgebung einen niedrigeren pH-Wert haben als gesunde Gewebe. So haben beispielsweise primäre Tumoren, Metastasen und Entzündungsherde in ihrer Umgebung erniedrigte pH-Werte. Die Liposomen der vorliegenden Erfindung können daher ihr verkapseltes Material, z.B. Antiinflammatorika oder Immunmodulatoren, spezifisch an den Entzündungsherd, z.B. die primären Tumore oder an Metastasen, transportieren und ihre Inhaltsstoffe in dieser sauren Umgebung freisetzen. Zum Beispiel können Liposomen, welche aus einer 3:7-Mischung von Dinatrium-N- [1,2-di-(9-cis-octadecenoyl)-sn-glycero-3-phosphoethanol]-N-hydroxysuccinylamin (I) und Dioleoylphosphatidylethanolamin bestehen, einen höheren Anteil ihrer Inhaltsstoffe, z.B. eine effektive Dosis rekombiniertes humanes Gamma Interferon, bei niedrigem pH, z.B. pH 4, abgeben als bei höheren oder neutralen pH-Werten. Dieser Befund kann *in-vitro* in Pufferlösungen mit einem pH von 7,4 und 4,0 nachgewiesen werden und zwar durch Bestimmung der Radioaktivität in regelmässigen Zeitabständen von markiertem $^{125}$I-Interferon, welches bei diesen pH-Werten von den Liposomen abgegeben wird. Beispielsweise werden mehr als 25% verkapseltes Gamma-Interferon bei einem pH-Wert von ca. 4 nach 180 Minuten von Liposomen abgegeben.

Daher sind die erfindungsgemässen pharmazeutischen Zusammensetzungen in Form von Liposomen ausgezeichnete Anwendungssysteme für die Abgabe von Wirkstoffen in die Umgebung von pathologischem Gewebe mit erniedrigten pH-Werten. Sie sind insbesondere in der Krebschemotherapie bei der Bekämpfung von metastasierenden Tumorzellen geeignet.

Wässrige Dispersionen, worin die Phospholipide der Formeln I und II in Form von Liposomen vorliegen, worin die genannten Verbindungen bzw. Stoffgemische mit biologischer Wirkung eingeschlossen sind, sind pharmazeutische Verabreichungssysteme, welche sich, gegebenenfalls nach Konzentrierung oder Isolierung der Liposomen, z.B. durch Ultrazentrifugieren, zu therapeutischen Zwecken für die parenterale (bukkal, lingual, sublingual, i.c., s.c., i.v., i.m., i.p. oder topisch) Verabreichung eignen.

Für die parenterale Verabreichung werden die Liposomen in einer sterilen wässrigen Lösung, welche als Trägerflüssigkeit dient, z.B. steriler, Calcium-freier, isotonischer Kochsalz- oder Glucoselösung, abgepuffert auf pH 7,0—7,8, vorzugsweise 7,2—7,4, dispergiert.

Für die topische Verabreichung wird die auf pH 7,0—7,8, vorzugsweise 7,2—7,4, abgepufferte Liposomen-haltige wässrige Dispersion mit üblichen festen Trägermaterialien, z.B. Verdicken, z.B. Hydroxypropylcellulose, sowie geeigneten Konservierungsmitteln, Antioxydanzien oder Duftstoffen vermischt und als Lotion oder Gel zur Applikation auf der Haut oder auf Schleimhäuten verwendet.

Die genannten pharmazeutischen Zusammensetzungen können als Trockenpräparate in den Handel gelangen und in Form eine wässrigen Liposomendispersion in einer auf pH 7,0—7,8 abgepufferten Trägerflüssigkeit appliziert werden.

Die Dosismenge für den zu applizierenden Wirkstoff ist im Allgemeinen die für den betreffenden Wirkstoff für die jeweilige Applikationsform, das Alter des Patienten und den gesundheitlichen Zustand des Patienten bekannte und z.B. im Deutschen Arzneimittelbuch (DAB), vorgeschriebene Höchst- und Mindestmenge. Wässrige Dispersionen mit erfindungsgemäss herstellbaren Liposomen haben aber auch den Vorteil, dass Wirkstoffe in geringeren Dosen appliziert zu den Rezeptoren gelangen und dort einen therapeutischen Effekt bewirken können oder bei Applikation von höheren Dosen unerwünschte Nebenwirkungen vermieden werden können.

Der bevorzugte Dosisbereich für Liposomen-verkapselte Immunmodulatoren des Muramylpeptid- oder Lipopeptidetyps beträgt etwa 0,001 bis 10 mg/kg Körpergewicht pro Dosis. Für humanes Gamma-Interferon oder Mischungen enthaltend MAF ist die bevorzugte Dosis ca. 0,01 ml Liposomendispersion pro Kilo Körpergewicht und 100—1000 Einheiten Gamma-Interferon oder MAF. Werden Muramylpeptide in Kombination mit Gamma-Interferon verabreicht, schätzt man die Höchstdosis für Menschen von ca. 70 kg Gewicht ca. 10 mg Liposomen pro Kilo Körpergewicht und 3 Mikrogramm Muramylpeptid und 1500 Einheiten Gamma-Interferon. Die höchste und die niedrigste Dosis des Einschlussmaterials sowie die Konzentration der Phospholipide in der Lösung können aufgrund von Resultaten von klinischen Versuchen variiert werden.

Die vorliegende Erfindung betrifft vorzugsweise pharmazeutische Zusammensetzungen in Form einer Liposomendispersion enthaltend

a) ein Phospholipid der Formel I, worin m zwei bedeutet, $R_1$ und $R_2$ weiter vorn definiert sind und X

$C_1$—$C_4$-Alkylen, $C_2$—$C_4$-Alkenylen oder $C_1$—$C_4$-Alkylen substituiert durch Hydroxy bedeuten oder ein pharmazeutisch annehmbares Salze davon,

b) ein Phospholipid der Formel II, worin $R_3$ und $R_4$ unabhängig voneinander geradkettiges $C_{10}$—$C_{20}$-Alkenoyl bedeuten,

c) eine Verbindung oder eine Kombination von Verbindungen mit pharmakologisch Eigenschaften und gegebenenfalls eines pharmazeutisch annehmbare Trägerflüssigkeit, welche auf pH 7,2—7,4 abgepuffert ist.

Die vorliegende Erfindung betrifft vorzugsweise pharmazeutische Zusammensetzungen in Form einer Liposomendispersion enthaltend

a) ein Phospholipid der Formel I, worin m zwei bedeutet, $R_1$ und $R_2$ weiter vorn definiert sind und X $C_2$—$C_4$-Alkylen oder $C_2$—$C_4$-Alkenylen sind, oder ein pharmazeutisch annehmbares Salz davon,

b) ein Phospholipid der Formel II, worin, $R_3$ und $R_4$ unabhängig voneinander geradkettiges $C_{10}$—$C_{20}$-Alkanoyl bedeuten,

c) eine Verbindung oder eine Kombination von Verbindungen aus der Gruppe der Antiphlogistika und/oder Antiinflammatorika, Antibiotika, Antileishmaniasis-Verbindungen, Antineoplastika und Immunmodulatoren und eine pharmazeutisch annehmbare Trägerflüssigkeit, welche auf pH 7,2—7,4 abgepuffert ist.

Die vorliegende Erfindung betrifft insbesondere pharmazeutische Zusammensetzungen in Form einer Liposomendispersion enthaltend

a) ein Phospholipid der Formel I, worin m zwei bedeutet, $R_1$ und $R_2$ unabhängig voneinander geradkettiges Alkanoyl oder Alkenoyl mit einer geraden Anzahl von 10 bis 20 C-Atomen und X $C_2$—$C_4$-Alkylen oder $C_2$—$C_4$-Alkenylen bedeuten, oder ein pharmazeutisch annehmbares Salz davon,

b) ein Phospholipid der Formel II, worin $R_3$ und $R_4$ geradkettiges $C_{10}$—$C_{20}$-Alkenoyl mit einer geraden Anzahl von 10 bis 20 C-Atomen bedeuten,

c) eine Verbindung oder eine Kombination von Verbindungen mit pharmakologischen Eigenschaften aus der Gruppe der Antiphlogistika und/oder Antiinflammatorika, Antibiotika, Antileishmaniasis-Verbindungen, Antineoplastika und Immunmodulatoren und eine pharmazeutisch annehmbare Trägerflüssigkeit, welche auf pH 7,2—7,4 abgepuffert ist.

Die Erfindung betrifft im engeren Sinne pharmazeutische Zusammensetzungen in Form einer Liposomendispersion enthaltend

a) ein Phospholipid der Formel I, worin m zwei bedeutet, $R_1$ und $R_2$ unabhängig voneinander 9-cis-Dodecenoyl, 9-cis-Tetradecenoyl, 9-cis-Hexadecenoyl, 6-cis-, 6-trans-, 9-cis-, 9-trans- oder 11-cis-Octadecenoyl oder 9-cis-Icosenoyl und X $C_2$—$C_4$-Alkenylen bedeuten, oder ein pharmazeutisch annehmbares Salz davon,

b) ein Phospholipid der Formel II, worin, $R_3$ und $R_4$ unabhängig voneinander 9-cis-Dodecenoyl, 9-cis-Tetradecenoyl, 9-cis-Hexadecenoyl, 6-cis-, 6-trans-, 9-cis-, 9-trans ode 11-cis-Octadecenoyl oder 9-cis-Icosenoyl bedeuten,

c) eine Verbindung oder eine Kombination von Verbindungen aus der Gruppe der Antiphlogistika und/oder Antiinflammatorika, Antibiotika, Antineoplastika und Immunmodulatoren und eine pharmazeutisch annehmbare Trägerlösung, welche auf pH 7,2—7,4 abgepuffert ist.

Insbesondere betrifft die vorliegende Erfindung pharmazeutische Zusammensetzungen in Form einer Liposomendispersion enthaltend

a) Natrium- oder Dinatrium-N-[1,2-di-(9-cis-octadecenoyl)-sn-glycero-3-phosphoethanol]-N-hydroxysuccinylamin oder Natrium- oder Dinatrium-N-[1,2-di-(9-cis-octadecenoyl)-sn-glycero-3-phosphoethanol]-N-hydroxyglutarylamin,

b) 1,2-Di-(9-cis-octadecenoyl)-sn-glycero-3-phosphoethanolamin,

c) eine Verbindung oder eine Kombination von Verbindungen aus der Gruppe der Antiphlogistika und/oder Antiinflammatorika, Antibiotika, Antineoplastika und Immunmodulatoren, und gegebenenfalls eine pharmazeutisch annehmbare Trägerflüssigkeit, welche auf pH 7,2—7,4 abgepuffert ist.

Besonders bevorzugt sind pharmazeutische Zusammensetzungen enthaltend

a) Natrium- oder Dinatrium-N-[1,2-di-(9-cis-octadecenoyl)-sn-glycero-3-phosphoethanol]-N-hydroxysuccinylamin oder Natrium- oder Dinatrium-N-[1,2-di-(9-cis-octadecenoyl)-sn-glycero-3-phosphoethanol]-N-hydroxyglutarylamin,

b) 1,2-Di-(9-cis-octadecenoyl)-sn-glycero-3-phosphoethanolamin,

c) eine Verbindung oder eine Kombination von Verbindungen aus der Gruppe Diclofenac, Pirprofen, Mitomycin, Cytarabin, Dactinomycin, Daunorubicin, Doxorubicin, Etoposid, N - Acetyl - D - muramyl - L - alanyl - D - iso - glutaminyl - L - alanin - 2 - [1,2 - dipalmitoyl - sn - glycero - 3 - hydroxyphosphoryloxy) - ethylamid, N - Acetyl - D - muramyl - L - alanyl - D - glutaminsäure - ($C^{\gamma}$ - L - alanin - 2 - (1,2 - dipalmitoyl - sn - glycero - 3 - hydroxyphosphoryloxy) - ethylamid-dinatriumsalz, N - Acetyl - D - muramyl - L - alanyl - D - isoglutamin - Natriumsalz, N - Acetyldesmethyl - muramyl - L - alanyl - D - isoglutamin - Natriumsalz, N - Acetyl - D - muramyl - L - alanyl - D - glutamin - α - n - butylester, $N^{\alpha}$ - (N - acetyl - D - muramyl - L - alanyl - D - isoglutaminyl) - $N^{\gamma}$ - stearoyl - L - lysin, 6 - O - stearoyl - N - acetyl - D - muramyl - L - alanin - D - isoglutamin und Lymphokine, und gegebenenfalls eine pharmazeutische annehmbare Trägerlösung, welche auf pH 7,2—7,4 abgepuffert ist.

Die vorliegende Erfindung hat ebenfalls Mischungen von Phospholipiden der Formeln I und II und

einer Verbindung oder Mischung von Verbindungen mit pharmakologischen Eigenschaften und gegebenenfalls einem Lipid aus der Gruppe der Phosphatidylcholine, Phosphatidylserin, Phosphatidylinositol, Phosphatidylglycerin, Cardiolipin und Cholesterin und Derivaten davon zum Gegenstand, insbesondere Mischungen, worin das molare Verhältnis der Phospholipidkomponenten a) zu b) (Phospholipide der Formel I und II) ungefähr 10 zu 90 bis 50 zu 50 beträgt. Die Mischungen lassen sich zur Herstellung von Liposomen in wässriger Phase, welche die Komponente c) (Wirkstoff oder Wirkstoffkombination) enthält, verwenden.

Die Erfindung betrifft ebenfalls ein Verfahren zur Herstellung der weiter vorn genannten pharmazeutischen Zusammensetzungen oder der genannten Mischungen, welches dadurch gekennzeichnet ist dass man

a) eine homogene Mischung bestehend aus den Phospholipiden der Formeln I und II und den genannten zusätzlichen Lipiden und einer lipophilen Verbindung oder Mischung von Verbindungen mit pharmakologischen Eigenschaften herstellt und zur Herstellung von Liposomen die erhältliche homogen Mischung in wässriger Phase dispergiert oder

b) eine homogene Mischung bestehend aus den Phospholipiden der Formeln I und II und den genannten zusätzlichen Lipiden herstellt und zur Herstellung von Liposomen die erhältliche homogene Mischung in wässriger Phase enthaltend eine wasserlösliche Verbindung oder Mischung von Verbindungen mit pharmakologischen Eigenschaften dispergiert und,

wenn notwendig, die erhältliche wässrige Dispersion auf pH 7,0—7,8 abpuffert und, wenn erwünscht, die erhältlichen Liposomen anreichert und/oder abtrennt.

Die Herstellung der homogen Mischung erfolgt beispielsweise durch Film- oder bevorzugt durch Lyophilisatbildung.

Die Filmbildung erfolgt nach Verfahrensvariante a) durch Auflösen der synthetischen Phospholipide der Formeln I und II und des lipophilen und zu verkapselnden Stoffs oder Stoffgemisches oder nach Verfahrensvariante b) durch Auflösen der synthetischen Phospholipide der Formeln I und II in einem organischen Lösungsmittel mit niedrigem Schmelzpunkt und Entfernen des Lösungsmittels.

Die Auswahl der geeigneten Lösungsmittel zur Herstsellung eines Films ist von der Löslichkeit der Lipidkomponenten und der Einschlussverbindungen abhängig. Geeignete Lösungsmittel zur Herstellung der homogenen Mischung durch Filmbildung sind beispielsweise niedrig siedende und schmelzende (unterhalb 0°), unsubstituierte oder substituierte, z.B. halogenierte, aliphatische oder cycloaliphatische Kohlenwasserstoffe, z.B. n-Hexan, Cyclohexan, Methylenchlorid oder Chloroform, Alkohole, z.B. Methanol, Niederalkancarbonsäureester, z.B. Essigsäureäthylester, oder Aether, z.B. Diäthyläther, oder Mischungen dieser Lösungsmittel. Man entfernt das Lösungsmittel im Vakuum, vorzugsweise Hochvakuum, oder durch Abblasen mit Inertgase, z.B. Stickstoff.

Die Lyophilisatbildung erfolgt nah Verfahrensvariante a) durch Lyophilisieren einer Lösung der synthetischen Phospholipide der Formeln I und II und des lipophilen zu verkapselnden Stoffs oder Stoffgemisches oder nach Verfahrensvariante b) durch Lyophilisieren einer Lösung der synthetischen Phospholipide der Formeln I und II in einem organischen Lösungsmittel mit hohem Schmelzpunkt auf die in der DE—A—2,818,655 beschriebene Weise. Geeignete Lösungsmittel liegen beim Gefriertrocknen, z.B. bei der Temperatur des Mathanol-, Aethanol- oder Aceton-Trockeneisgemisches, zusammen mit den Lipidkomponenten und den Einschlussverbindungen in festem Zustand vor und sind z.B. organische Lösungsmittel mit einem Schmelzpunkt höher als 0°C, z.B. Eisessig, Benzol oder Dioxan, insbesondere tert-Butanol.

Eine homogene Mischung lässt sich auch durch Sprühtrocknung einer Lösung der Phospholipide und der lipophilen Einschlussverbindungen in einem organischen Lösungsmittel, z.B. Chloroform, herstellen. Man erhält die homogene Mischung in Form eines Pulvers.

Zur Herstellung der homogenen Mischung eignet sich ein ungefähres Mischungsverhältnis der Phospholipide der Formel I zu Phospholipiden der Formel II ca. 10 zu 90 bis ca. 50 zu 50 Molprozent, insbesondere 30 zu 70 Molprozent. Das ungefähre Mischungsverhältnis der Einschlussverbindungen zur Gesamtmenge der Lipide beträgt ca. 0,001 bis 1,0 zu 1,0, bevorzugt 0,005 bis 0,1 zu 1,0 Mol.

Man dispergiert beispielsweise durch Schütteln (z.B. Vortex-Mischer) oder Rühren der wässrigen Phase, der man nach Verfahrensvariante a) die zuvor hergestellte homogene Mischung der Phospholipide (I) und (II) und der lipophilen Einschlussverbindungen zugesetzt hat, oder welche nach Verfahrensvariante b) die wasserlösliche Einschlussverbindung enthält und der man die zuvor hergestellte homogene Mischung der Phospholipide (I) und (II) zugesetzt hat. Die Bildung von Liposomen, welche gross, klein, unilamellar oder multilamellar sein können, findet spontan (spontaneous vesiculation), d.h. ohne zusätzliche Energiezufuhr von aussen und mit grosser Geschwindigkeit statt. Es können ca. 0,1 bis 50 Gewichtsprozent, vorzugsweise 2 bis 20 Gewichtsprozent (bezogen auf das Gesamtgewicht der wässrigen Dispersion), der homogenen Mischung in wäsriger Phase dispergiert werden.

Man puffert sauer oder basisch reagierende wässrige Dispersionen auf pH 7,0—7,8, vorzugsweise pH 7,2—7,4, ab. Vorzugsweise dispergiert man in einer bereits auf diesen pH-Wert abgepufferten, wässrigen Phase.

Die Verfahrensvariante a) eignet sich besonders, wenn lipophile und schlecht wasserlösliche Wirkstoffe in Form von Liposomen eingeschlossen werden sollen, z.B. Muramyldi- oder Muramyltripeptide. Die Verfahrensvariante b) bietet sich an, wenn eine Liposomenmischung aus

wasserlöslichen Wirkstoffen, z.B. Cytarabin oder Zytostatika vom Typ Trifosfamid, hergestellt werden soll.

Die Herstellung der erfindungsgemässen pharmazeutischen Zusammensetzungen in Form von wässrigen Liposomen-Mischungen kann auch nach sämtlichen anderen bisher bekannt gewordenen Verfahren zur Herstellung von Liposomen erfolgen, z.B. auf herkömmliche Weise durch Beschallung mit Ultraschall der wässrigen Dispersion enthaltend die Phospholipide (I) und (II) und die Einschlussverbindungen, durch Infusionsmethoden oder durch Umkehrphasen-Verdampfung.

Man dispergiert bei Temperaturen unterhalb ca. 60°C, vorzugsweise bei Raumtemperatur. Falls es die Empfindlichkeit der zu verkapselnden Verbindungen verlangt, führt man das Verfahren unter Kühlen und gegebenenfalls Inertgasatmosphäre, z.B. Stickstoff- oder Argonatmosphäre, durch. Die erhältlichen Liposomen sind in wässriger Phase sehr lange (bis zu mehreren Wochen oder Monaten) beständig. Pharmazeutische Zusammensetzungen in Form von wässrigen Liposomendispersionen können gegebenenfalls nach Zusatz von Stabilisatoren, z.B. Mannit oder Lactose, durch Gefriertrocknung lagerfähig gemacht werden.

Die Grösse der gebildeten unilamellaren Liposomen ist u.a. von der Struktur des Wirkstoffs und der Lipidkomponenten, dem Mischungs verhältnis der Komponenten und der Konzentration dieser Komponenten in der wässrigen Dispersion abhängig. So kann man beispielsweise durch erhöhung oder Erniedrigung der Konzentration der Lipidkomponenten wässrige Phasen mit einem hohen Anteil an kleinen oder grossen Liposomen herstellen.

In wässriger Phase befindliche Liposomen mit einem Durchmesser grösser als $6{,}0 \times 10^{-8}$m, z.B. grosse multilamellare Liposomen, können durch Gelfiltration, z.B. mit Sepharose oder mit Sephacryl als Träger, abgetrennt werden.

Durch Extrusion durch geradporige Filter, z.B. Membranfilter des Acrodisc®, Nucleopore® oder Polycarbonat-Typs mit einem Porendurchmesser von ca. $1{,}0 \times 10^{-6}$—$1{,}0 \times 10^{-8}$m bei einem Druck von ca. 0,1 bis 1,5 bar und einer Filtrationsgeschwindigkeit von ca. 20 ml/h können Liposomen mit einer besonders einheitlichen Grössenverteilung erhalten werden.

Liposomen werden vorzugsweise abgetrennt, wenn gemäss Verfahrensvariante b) die wässrige Phase nicht verkapselte, wasserlösliche Verbindungen enthält. Insbesondere sollten wasserlösliche Antineoplastika, z.B. Alkylanzien, beispielsweise durch Filtration, Ultrafiltration, Dialyse oder Zentrifugieren abgetrennt werden, da die Verträglichkeit dieser Wirkstoffe in gelöster Form nicht besonders gut ist. Die angereicherten Liposomen können mit einer Trägerflüssigkeit, z.B. isotonischer, steriler Kochsalzlösung, vermischt werden. Wässrige Dispersionen mit kleinen Liposomen von relative einheitlicher Grösse erhält man auch durch Behandlung mit Ultraschall.

Die erfolgte Bildung von Liposomen und ihr Gehalt in wässriger Phase lässt sich in an sich bekannter Weise anhand verschiedener physikalischer Messmethoden nachweisen, z.B. mit gefriergebrochenen (freeze fracture) Proben und Dünnschnitten im Elektronenmikroskop oder durch Röntgendiffraktion, durch dynamische Lichtstreuung, durch Massenbestimmung des Filtrates in der analytischen Ultrazentrifuge und vor allem spektroskopisch, z.B. im Kernresonanzspektrum ($^{1}H$, $^{13}C$ und $^{31}P$).

Synethetische Phospholipide der Formel I sind bekannt. Die Herstellung dieser Verbindungen und ihre Verwendung als Zwischenprodukte ist in der Europäischen Patentanmeldung Nr. 56992 beschrieben.

Phosphorlipide der Formel II sind bekannt. Einige sind kommerziell erhältlich, z.B. bei den Firmen Avanti, Fluka, Serva, Sigma.

Die Herstellung von Muramylpeptiden der Formel V ist in der Britischen Patentschrift 1,570,625 und in den Europäischen Patentanmeldungen 25,495 und 21,367 beschrieben. Immunmodulatoren des Lipopeptid-Taps sind ebenfalls bekannt, siehe Europäische Patentanmeldung 11,787 und Europäische Patentschrift 330.

Die Herstellung von gereinigtem, natürlichem oder rekombiniertem Gamma-Interferon ist in den Europäischen Patentanmeldungen 63,482, 77,670, 83,540, 89,676, 95,350, 99,084, 110,044, 112,976 oder 121,157, in der Britischen Patentschrift 2,107,718 und in den Internationalen Anmeldungen WO 83/04053 beschrieben.

Die Herstellung von gereinigtem Interleukin 2 ist in der Europäischen Patentanmeldung 106,179 und in der US-Patentschrift 4,448,879 beschrieben.

Die verwendeten Pufferlösungen vom pH 7,0—7,8 sind vorzugsweise sterile Phosphatpufferlösungen auf der Basis Dihydrogen-/ Hydrogenphosphat, welche beispielsweise nach der in Hagers Handbuch der Pharmazeutischen Praxis, Springer Verlag, band 1 S. 357—359 gegebenen Vorschrift hergestellt werden können. Insbesondere wird sterile, isotonische Calcium-freie Pufferlösung von pH 7.2 (Dulbecco) verwendet.

Die nachfolgenden Beispiele veranschaulichen die Erfindung ohne sie zu beschränken. Temperaturen sind in Graden Celsius angegeben.

## Beispiel 1

a) In einem Rundkolben werden 84,70 mg (0,098 mMol) Dinatrium - N - [1,2 - di - (9 - cis - octadecenoyl) - sn - glycero - 3 - phosphoethanol] - N - hydroxysuccinylamin und 168,10 mg (0.226 mMol) 1,2 - Di - (9 - cis - octadecenoyl) - sn - glycero - 3 - phosphoethanolamin in einer ausreichenden Menge tert-Butanol gelöst, bis sich beide Lipide vollständig lösen. Die Lösung wird unter sterilen Bedingungen über einem Acrodisc®-Filter ($2{,}0 \times 10^{-7}$m) filtriert und in einer sterilen Viale abgefüllt.

Die Viale wird auf −45° eingefroren, ein Vakuum angelegt und das Lösungsmittel entfernt, bis Raumtemperatur erreicht ist. Die Viale wird unter Argon-Schutzgasatmosphäre verschlossen.

Zu dieser Viale enthaltend ein Lyophilisat der genannten Lipidkomponenten werden 2,5 ml sterile, Phosphat-gepufferte (pH 7,2—7,4), Calcium-freie Natriumchloridlösung (Dulbecco) enthaltend Doxorubicin in einer Konzentration von 4 g/l mit einer sterilen Spritze gegeben. Die Viale wird anschliessend 10 Minuten lang auf einem standardisierten Labormischer (Vortex, Stufe 6) gemischt und zentrifugiert. Nach 60 Minuten langem Zentrifugieren in einem Schwerefeld von ca. 40,000 × g wird die überstehende Lösung dekantiert. Der Liposomenrückstand in disperser Form wird wiederum in 2,5 ml steriler, Phosphat-gepufferter (pH 7,2—7,4), Calcium-freier Natriumchlorid-Lösung suspendiert. Zentrifugieren und nochmalige Suspendierung werden nach Bedarf wiederholt, bis sich in der überstehenden Lösung kein Doxorubicin mehr befindet. Die hergestellte Liposomendispersion lässt sich parenteral applizieren.

Beispiel 2

Analog Beispiel 1 werden wässrige Liposomendispersionen erhalten, indem man 84,70 mg (0,098 mMol) Dinatrium - N - [1,2 - di - (9 - cis - octadecenoyl) - sn - glycero - 3 - phosphoethanol] - N - hydroxysuccinylamin und 168,10 mg (0,226 mMol) 1,2 - Di - (9 - cis - octadecenoyl) - sn - glycero - 3 - phosphoethanolamin mit 0,1 mg bis 10 mg N - Acetyl - L - muramyl - L - alanyl - D - isoglutamin - natriumsalz oder 0,1 mg bis 10 mg N - Acetyl desmethylmuramyl - L - alanyl - D - isoglutamin - natriumsalz oder 1.000 bis 100.000 Einheiten rekombiniertem, humanem Gamma-Interferon erhältlich gemäss EP—A—121,157 (Kyowa Hakko Kogyo Co.) oder mit einer Kombination von 1.000 bis 100.000 einheiten dieses rekombinierten, humanen Gamma-Interferons mit 50—200 Mikrogramm N - Acetyl - L - muramyl - L - alanyl - D - isoglutamin - natriumsalz oder N - Acetyldesmethylmuramyl - L - alanyl - D - isoglutamin - natriumsalz dispergiert.

Beispiel 3

a) In einem Rundkolben werden 84,70 mg (0,098 mMol Dinatrium - N - [1,2 - di - (9 - cis - octadecenoyl) - sn - glycero - 3 - phosphoethanol] - N - hydroxysuccinylamin und 168, 10 mg (0,226 mMol) 1,2 - Di - (9 - cis - octadecenoyl) - sn - glycero - 3 - phosphoethanolamin in einer ausreichenden Menge tert-Butanol gelöst, bis sich beide Lipide vollständig lösen. Die Lösung wird unter sterilen Bedingungen über einem Acrodisc®-Filter (2,0 × $10^{-7}$ m) filtriert und in einer sterilen Viale abgefüllt. Die Viale wird bei 1750 rpm (rotations per minute) rotiert und das Lösungsmittel in einem Strom von gereinigtem, filtriertem (bei 1 bar), trockenem Stickstoff entfernt. Die Viale wird im Vakuum bei 6,0 × $10^{-2}$ mbar evakuiert und der Inhalt anschliessend unter Argon aufbewahrt.

Zu dieser Viale enthaltend einen dünnten Film mit den genannten Lipidkomponenten werden 2,5 ml sterile, Phosphat-gepufferte (pH 7,2—7,4), Calcium-freie Natriumchloridlösung (Dulbecco) enthaltend Diclofenac-Natrium in einer Konzentration von 7 g/l mit einer sterilen Spritze gegeben. Die Viale wird anschliessend 10 Minuten lang auf einem stardardisierten Labormischer (Vortex, Stufe 6); gemischt und zentrifugiert. Nach 60 Minuten langem Zentrifugieren in einem Schwerefeld von ca. 40,000 × g wird die überstehende Lösung dekantiert. Der Liposomenrückstand in disperser Form wird wiederum in 2,5 ml steriler, Phosphat-gepufferter (pH 7,2—7,4), Calcium-freier Natriumchlorid-Lösung suspendiert. Die hergestellte Liposomendispersion lässt sich parenteral applizieren.

Beispiel 4

In einem Rundkolben werden 0,1 g N - Acetylmuramyl - L - alanyl - D - iso - glutaminyl - L - alanin - 2 - (1,2 - dipalmitoyl - sn - glycero - 3 - hydroxyphosphoryloxy) - ethylamid (Herstellung siehe Europäische Patentschrift 25,495), 84,7 mg (0,098 mMol) Dinatrium - N - [1,2 - di - (9 - cis - octadecenoyl) - sn - glycero - 3 - phosphoethanol] - N - hydroxysuccinylamin und 168,10 mg (0,226 mMol) 1,2 - Di - (9 - cis - octadecenoyl) - sn - glycero - 3 - phosphoethanolamin in einer so grossen Menge tert-Butanol gelöst, bis sich beide Lipide vollständig lösen. Die Lösung wird unter sterilen Bedingungen über einem Acrodisc®-Filter (2,0 × $10^{-7}$m) filtriert und in einer sterilen Viale abgefüllt. Die Viale wird bei 1750 rpm rotiert und das Lösungsmittel in einem Strom aus gereinigtem, filtriertem (bei 1 bar), trockenem Stickstoff entfernt. Die Viale wird im Vakuum bei 6,0 × $10^{-2}$ mbar evakuiert und der Inhalt anschliessend unter Argon aufbewahrt.

Zu dieser Viale enthaltend einen dünnen Film mit den genannten Lipidkomponenten werden 2,5 ml sterile, Phosphat-gepufferte (pH 7,2—7,4) Calcium-freie Natriumchloridlösung (Dulbecco) mit einer sterilen Spritze hinzugegeben. Die Viale wird anschliessend zehn Minuten lang auf einem standardisierten Labormischer (Vortex, Stufe 6) gemischt und zentrifugiert.

Die Liposomendispiersion ist bei 4° lagerfähig und kann für die parenterale Applikation verwendet werden.

Beispiel 5

In einem Rundkolben werden 0,1 g N - Acetyl - D - muramyl - L - alanyl - D - isoglutaminyl - L - alanin - 2 - (1,2 - dipalmitoyl - sn - glycero - 3 - hydroxyphosphoryloxy) - ethylamid (Herstellung siehe Europäische Patentschrift 25,495), 84,7 mg (0,098 mMol) Dinatrium - N - [1,2 - di - (9 - cis - octadecenoyl) - sn - glycero - 3 - phosphoethanol] - N - hydroxysuccinylamin und 168,10 mg (0,226 mMol) 1,2 - Di - (9 - cis - octadecenoyl) - sn - glycero - 3 - phosphoethanolamin in einer so grossen Menge

tert-Butanol gelöst, bis sich beide Lipide vollständig lösen. Die Lösung wird unter sterilen Bedingungen über einem Acrodisc®-Filter (2,0 × $10^{-7}$m) filtriert und in einer sterilen Viale abgefüllt. Die Viale wird auf −45° eingefroren, ein Vakuum angelegt und das Lösungsmittel entfernt, bis Raumtemperatur erreicht ist. Die Viale wird unter Argon-Schutzgasatmosphäre verschlossen.

Zu dieser Viale enthaltend ein Lyophiliat mit den genannten Lipidkomponenten werden 2,5 ml sterile, Phosphat-gepufferte (pH 7,2—7,4) Calcium-freie Natriumchloridlösung (Dulbecco) mit einer sterilen Spritze hinzugegeben. Die Viale wird anschliessend zehn Minuten lang auf einem standardisierten Labormischer (Vortex, Stufe 6) gemischt und zentrifugiert.

Die Liposomendispersion ist bei 4° lagerfähig und kann für die parenterale Applikation verwendet werden.

Beispiel 6

Analog Beispiel 4 oder 5 kann man Liposomendispersionen enthaltend 0,1 mg bis 10 mg N - Acetylmuramyl- - L - alanyl - D - isoglutaminyl - L - alanin - 2 - (1,2 - dipalmitoyl - sn - glycero - 3 - hydroxyphosphoryloxy) - ethylamid, 84.70 mg (0.098 mmol) Dinatrium - N - [1,2 - di - (9 - cis - octadecenoyl) - sn - glycero - 3 - phosphoethanol] - N - hydroxysuccinylamin und 168.10 mg (0,226 mMol) 1,2 - Di - (9 - cis - octadecenoyl) - sn - glycero - 3 - phosphoethanolamin herstellen.

Beispiel 7

Analog Beispiel 1 kann man Liposomendispersionen enthaltend 86,3 mg (0,098 mmol) Dinatrium - N - [1,2 - di - (9 - cis - octadecenoyl) - sn - glycero - 3 - phosphoethanol] - N - hydroxysuccinylamin und 168,1 mg (0,026 mmol) 1,2 - Di - (9 - cis - octadecenoyl) - sn - glycero - 3 - phosphoethanolamin und 0,1 mg bis 10 mg N - Acetyl - L - muramyl - L - alanyl - D - isoglutamin - natriumsalz oder 0,1 mg bis 10 mg N - acetyl - desmethylmuramyl - L - alanyl - D - isoglutamin-natriumsalz herstellen.

**Patentansprüche für die Vertragsstaaten BE CH DE FR GB IT LI LU NL SE**

1. Pharmazeutische Zusammensetzungen in Form einer Liposomendispersion bestehend aus

a) einem Phospholipid der Formel

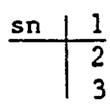
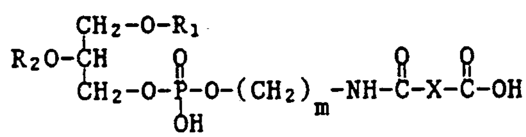
(I),

worin m zwei oder drei, $R_1$ und $R_2$ unabhängig voneinander Alkyl, Alkenyl oder Acyl mit je 10 bis 20 C-Atomen und X die direkte Bindung, $C_1$—$C_4$-Alkylen, $C_2$—$C_4$-Alkenylen oder $C_1$—$C_4$-Alkylen substituiert durch Hydroxy bedeuten, oder ein pharmazeutisch annehmbares Salz davon,

b) einem Phospholipid der Formel

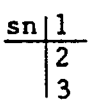
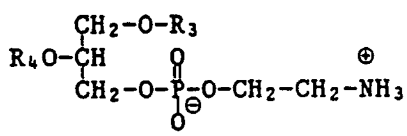
(II),

worin $R_3$ und $R_4$ die Acylgruppe einer gesättigten oder ungesättigten Carbonsäure mit 10—20 C-Atomen und 1—2 Doppelbindungen bedeuten,

c) einer Verbindung oder einer Mischung von Verbindungen mit pharmakologischen Eigenschaften und gegebenenfalls

d) einem Lipid aus der Gruppe Phosphatidylcholin, Phosphatidylserin, Phosphatidylinositol, Phosphatidylglycerin, Cardiolipin und Cholesterin und gegebenenfalls einer pharmazeutisch annehmbaren Trägerlösung, welche auf pH 7,0—7,8 gepuffert ist, und gegebenenfalls pharmazeutisch annehmbaren Hilfsstoffen.

2. Pharmazeutische Zusammensetzungen gemäss Anspruch 1 enthaltend

a) ein Phospholipid der Formel I, worin m zwei bedeutet, $R_1$ und $R_2$ weiter vorn definiert sind und X $C_1$—$C_4$-Alkylen, $C_2$—$C_4$-Alkenylen oder $C_2$—$C_4$-Alkenylen substituiert durch Hydroxy bedeutet oder ein pharmazeutisch annehmbares Salze davon,

b) ein Phospholipid der Formel II, worin $R_3$ und $R_4$ unabhängig voneinander geradkettiges $C_{10}$—$C_{20}$-Alkenoyl bedeuten,

c) eine Verbindung oder eine Kombination von Verbindungen mit pharmakologischen Eigenschaften und gegebenenfalls eine pharmazeutisch annehmbare Trägerflüssigkeit, welche auf pH 7,2—7,4 abgepuffert ist.

3. Pharmazeutische Zusammensetzungen gemäss Anspruch 1 enthaltend

a) ein Phospholipid der Formel I, worin m zwei bedeutet, $R_1$ und $R_2$ weiter vorn definiert sind und X $C_2$—$C_4$-Alkylen oder $C_2$—$C_4$-Alkenylen sind, oder ein pharmazeutisch annehmbares Salz davon,

b) ein Phospholipid der Formel II, worin $R_3$ und $R_4$ unabhängig voneinander geradkettiges $C_{10}$—$C_{20}$-Alkanoyl bedeuten,

c) eine Verbindung oder eine Kombination von Verbindungen aus der Gruppe der Antiphlogistika und/oder Antiinflammatorika, Antibiotika, Antileishmaniasis-Verbindungen, Antineoplastika und Immunmodulatoren und gegebenenfalls eine pharmazeutisch annehmbare Trägerflüssigkeit, welche auf pH 7,2—7,4 abgepuffert ist.

4. Pharmazeutische Zusammensetzungen gemäss Anspruch 1 in Form einer Liposomendispersion enthaltend

a) ein Phospholipid der Formel I, worin m zwei bedeutet, $R_1$ und $R_2$ unabhängig voneinander geradkettiges Alkanoyl oder Alkenoyl mit einer geraden Anzahl von 10 bis 20 C-Atomen und X $C_2$—$C_4$-Alkylen oder $C_2$—$C_4$-Alkenylen bedeuten, oder ein pharmazeutisch annehmbares Salz davon,

b) ein Phospholipid der Formel II, worin, $R_3$ und $R_4$ geradkettiges $C_{10}$—$C_{20}$-Alkenoyl mit einer geraden Anzahl von 10 bis 20 C-Atomen bedeuten,

c) eine Verbindung oder eine Kombination von Verbindungen mit pharmakologischen Eigenschaften aus der Gruppe der Antiphlogistika und/oder Antiinflammatorika, Antibiotika, Antileishmaniasis-Verbindungen, Antineoplastika und Immunmodulatoren und eine pharmazeutisch annehmbare Trägerflüssigkeit, welche auf pH 7,2—7,4 abgepuffert ist.

5. Pharmazeutische Zusammensetzungen gemäss Anspruch 1 in Form einer Liposomendispersion enthaltend

a) ein Phospholipid der Formel I, worin m zwei bedeutet, $R_1$ und $R_2$ unabhängig voneinander 9-cis-Dodecenoyl, 9-cis-Tetradecenoyl, 9-cis-Hexadecenoyl, 6-cis-, 6-trans-, 9-cis-, 9-trans- oder 11-cis-Octadecenoyl oder 9-cis-Icosenoyl und X $C_2$—$C_4$-Alkylen oder $C_2$—$C_4$-Alkenylen bedeuten, oder ein pharmazeutisch annehmbares Salz davon,

b) ein Phospholipid der Formel II, worin $R_3$ und $R_4$ unabhängig voneinander 9-cis-Dodecenoyl, 9-cis-Tetradecenoyl, 9-cis-Hexadecenoyl, 6-cis-, 6-trans-, 9-cis-, 9-trans oder 11-cis-Octadecenoyl oder 9-cis-Icosenoyl bedeuten,

c) eine Verbindung oder eine Kombination von Verbindungen aus der Gruppe der Antiphlogistika und/oder Antiinflammatorika, Antibiotika, Antineoplastika und Immunmodulatoren und eine pharmazeutisch annehmbare Trägerflüssigkeit, welche auf pH 7,2—7,4 abgepuffert ist.

6. Pharmazeutische Zusammensetzungen gemäss Anspruch 1 in Form einer Liposomendispersion enthaltend

a) Natrium- oder Dinatrium-N-[1,2-di-(9-cis-octadecenoyl)-sn-glycero-3-phosphoethanol]-N-hydroxysuccinylamin oder Natrium- oder Dinatrium-N-[1,2-di-(9-cis-octadecenoyl)-sn-glycero-3-phosphoethanol]-N-hydroxyglutarylamin,

b) 1,2-Di-(9-cis-octadecenoyl)-sn-glycero-3-phosphoethanolamin,

c) eine Verbindung oder eine Kombination von Verbindungen aus der Gruppe der Antiphlogistika und/oder Antiinflammatorika, Antibiotika, Antineoplastika und Immunmodulatoren, und eine pharmazeutisch annehmbare Trägerflüssigkeit, welche auf pH 7,2—7,4 abgepuffert ist.

7. Pharmazeutische Zusammensetzungen gemäss Anspruch 1 in Form einer Liposomendispersion enthaltend

a) Natrium- oder Dinatrium-N-[1,2-di-(9-cis-octadecenoyl)-sn-glycero-3-phosphoethanol]-N-hydroxysuccinylamin oder Natrium- oder Dinatrium-N-[1,2-di-(9-cis-octadecenoyl)-sn-glycero-3-phosphoethanol]-N-hydroxyglutarylamin,

b) 1,2-Di-(9-cis-octadecenoyl)-sn-glycero-3-phosphoethanolamin,

c) eine Verbindung oder Kombination von Verbindungen aus der Gruppe Diclofenac, Pirprofen, Mitomycin, Cytarabin, Dactinomycin, Daunorubicin, Doxorubicin, Etoposid, N - Acetyl - D - muramyl - L - alanyl - D - isoglutaminyl - L - alanin - 2 - [1,2 - dipalmitoyl - sn - glycero - 3 - hydroxyphosphoryloxy) - ethylamid, N - Acetyl - D - muramyl - L - alanyl - D - glutaminsäure - ($C^{\gamma}$ - L - alanin - 2 - (1,2 - dipalmitoyl - sn - glycero - 3 - hydroxyphosphoryloxy) - ethylamid) - dinatriumsalz, N - Acetyl - D - muramyl - L - alanyl - D - isoglutamin - natriumsalz, N - Acetyl - D - muramyl - L - alanyl - D - glutamin - α - n - butylester, $N^{\alpha}$ - (N - acetyl - D - muramyl - L - alanyl - D - isoglutaminyl) - $N^{\gamma}$ - stearoyl - L - lysin, 6 - O - stearoyl - N - acetyl - D - muramyl - L - alanin - D - isoglutamin und Lymphokin, und eine pharmazeutisch annehmbare Trägerflüssigkeit, welche auf pH 7,2—7,4 abgepuffert ist.

8. Mischungen geeignet zur Herstellung von Liposomen bestehend aus Phospholipiden der Formeln I und II und einer Verbindung oder Mischung von Verbindungen mit pharmakologischen Eigenschaften gemäss Anspruch 1.

9. Verfahren zur Herstellung von pharmazeutischen Zusammensetzungen gemäss einem der Ansprüche 1—7, dadurch gekennzeichnet, dass man

a) eine homogene Mischung bestehend aus den Phospholipiden der Formeln I und II und den genannten zusätzlichen Lipiden und einer lipophilen Verbindung oder Mischung von Verbindungen mit pharmakologischen Eigenschaften herstellt und zur Herstellung von Liposomen die erhältliche homogene Mischung in wässriger Phase dispergiert oder

b) eine homogene Mischung bestehend aus den Phospholipiden der Formeln I und II und den genannten zusätzlichen Lipiden herstellt und zur Herstellung von Liposomen die erhältliche homogene

Mischung in wässriger Phase enthaltend eine wasserlösliche Verbindung oder Mischung von Verbindungen mit pharmakologischen Eigenschaften dispergiert und,

wenn notwendig, die erhältliche wässrige Dispersion auf pH 7,0—7,8 abpuffert und, wenn erwünscht, die erhältlichen Liposomen anreichert und/oder abtrennt.

10. Verabreichungssysteme gemäss Anspruch 1 zur Anwendung bei der Behandlung des menschlichen oder tierischen Körpers.

**Patentansprüche für den Vertragsstaat AT**

1. Verfahren zur Herstellung von pharmazeutischen in Form einer Liposomendispersion Zusammensetzungen bestehend aus

a) einem Phospholipid der Formel

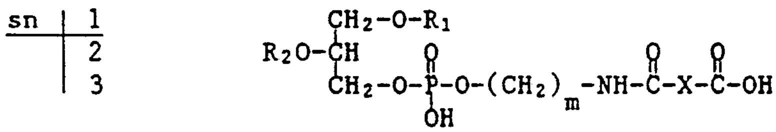

(I),

worin m zwei oder drei, $R_1$ und $R_2$ unabhängig voneinander Alkyl, Alkenyl oder Acyl mit je 10 bis 20 C-Atomen und X die direkte Bindung, $C_1$—$C_4$-Alkylen, $C_2$—$C_4$-Alkenylen oder $C_1$—$C_4$-Alkylen oder $C_2$—$C_4$-Alkenyl substituiert durch Hydroxy bedeuten, oder ein pharmazeutisch annehmbares Salz davon,

b) einem Phospholipid der Formel

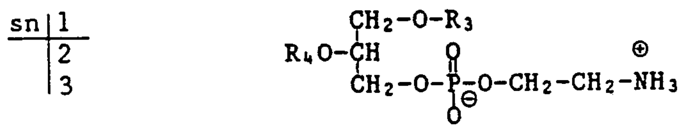

(II),

worin $R_3$ und $R_4$ die Acylgruppe einer gesättigten oder ungesättigten Carbonsäure mit 10—20 C-Atomen und 1—2 Doppelbindungen bedeuten,

c) einer Verbindung oder einer Mischung von Verbindungen mit pharmakologischen Eigenschaften und gegebenenfalls

d) einem Lipid aus der Gruppe Phosphatidylcholin, Phosphatidylserin, Phosphatidylinositol, Phosphatidylglycerin, Cardiolipin und Cholesterin und einer pharmazeutisch annehmbaren Trägerlösung, welche auf pH 7,0—7,8 gepuffert ist, und gegebenenfalls pharmazeutisch annehmbaren Hilfsstoffen, dadurch gekennzeichnet, dass man

a) eine homogene Mischung bestehend aus den Phospholipiden der Formeln I und II und den genannten zusätzlichen Lipiden und einer lipophilen Verbindung oder Mischung von Verbindungen mit pharmakologischen Eigenschaften herstellt und zur Herstellung von Liposomen die erhältliche homogene Mischung in wässriger Phase dispergiert oder

b) eine homogene Mischung bestehend aus den Phospholipiden der Formeln I und II und den genannten zusätzlichen Lipiden herstellt und zur Herstellung von Liposomen die erhältliche homogene Mischung in wässriger Phase enthaltend eine wasserlöslichen Verbindung oder Mischung von Verbindungen mit pharmakologischen Eigenschaften dispergiert und,

wenn notwendig, die erhältliche wässrige Dispersion auf pH 7,0—7,8 abpuffert und, wenn erwünscht, die erhältlichen Liposomen anreichert und/oder abtrennt.

2. Verfahren zur Herstellung von pharmazeutischen Zusammensetzungen gemäss Anspruch 1 in Form einer Liposomendispersion enthaltend

a) ein Phospholipid der Formel I, worin m zwei bedeutet, $R_1$ und $R_2$ weiter vorn definiert sind und X $C_1$—$C_4$-Alkylen, $C_2$—$C_4$-Alkenylen oder $C_1$—$C_4$-Alkylen substituiert durch Hydroxy bedeuten oder ein pharmazeutisch annehmbares Salz davon,

b) ein Phospholipid der Formel II, worin $R_3$ und $R_4$ unabhängig voneinander geradkettiges $C_{10}$—$C_{20}$-Alkenoyl bedeuten,

c) eine Verbindung oder eine Kombination von Verbindungen mit pharmakologischen Eigenschaften und eine pharmazeutisch annehmbare Trägerflüssigkeit, welche auf pH 7,2—7,4 abgepuffert ist.

3. Verfahren zur Herstellung von pharmazeutischen Zusammensetzungen gemäss Anspruch 1 in Form einer Liposomendispersion enthaltend

a) ein Phospholipid der Formel I, worin m zwei bedeutet, $R_1$ und $R_2$ weiter vorn definiert sind und X $C_2$—$C_4$-Alkylen oder $C_2$—$C_4$-Alkenylen sind, oder ein pharmazeutisch annehmbares Salz davon,

b) ein Phospholipid der Formel II, worin $R_3$ und $R_4$ unabhängig voneinander geradkettiges $C_{10}$—$C_{20}$-Alkanoyl bedeuten,

c) eine Verbindung oder eine Kombination von Verbindungen aus der Gruppe der Antiphlogistika und/oder Antiinflammatorika, Antibiotika, Antileishmaniasis-Verbindungen, Antineoplastika und Immunmodulatoren und eine pharmazeutisch annehmbare Trägerflüssigkeit, welche auf pH 7,2—7,4 abgepuffert ist.

4. Verfahren zur Herstellung von pharmazeutischen Zusammensetzungen gemäss Anspruch 1 in Form einer Liposomendispersion enthaltend

a) ein Phospholipid der Formel I, worin m zwei bedeutet, $R_1$ und $R_2$ unabhängig voneinander geradkettiges Alkanoyl oder Alkenoyl mit einer geraden Anzahl von 10 bis 20 C-Atomen und X $C_2$—$C_4$-Alkylen oder $C_2$—$C_4$-Alkenylen bedeuten, oder ein pharmazeutisch annehmbares Salz davon,

b) ein Phospholipid der Formel II, worin, $R_3$ und $R_4$ geradkettiges $C_{10}$—$C_{20}$-Alkenoyl mit einer geraden Anzahl von 10 bis 20 C-Atomen bedeuten,

c) eine Verbindung oder eine Kombination von Verbindungen mit pharmakologischen Eigenschaften aus der Gruppe der Antiphlogistika und/oder Antiinflammatorika, Antibiotika, Antileishmaniasis-Verbindungen, Antineoplastika und Immunmodulatoren und eine pharmazeutisch annehmbare Trägerflüssigkeit, welche auf pH 7,2—7,4 abgepuffert ist.

5. Verfahren zur Herstellung pharmazeutischer Zusammensetzungen gemäss Anspruch 1 in Form einer Liposomendispersion enthaltend

a) ein Phospholipid der Formel I, worin m zwei bedeutet, $R_1$ und $R_2$ unabhängig voneinander 9-cis-Dodecenoyl, 9-cis-Tetradecenoyl, 9-cis-Hexadecenoyl, 6-cis-, 6-trans-, 9-cis-, 9-trans- oder 11-cis-Octadecenoyl oder 9-cis-Icosenoyl und X $C_2$—$C_4$-Alkylen oder $C_2$—$C_4$-Alkenylen bedeuten, oder ein pharmazeutisch annehmbares Salz davon,

b) ein Phospholipid der Formel II, worin $R_3$ und $R_4$ unabhängig voneinander 9-cis-Dodecenoyl, 9-cis-Tetradecenoyl, 9-cis-Hexadecenoyl, 6-cis-, 6-trans-, 9-cis-, 9-trans oder 11-cis-Octadecenoyl oder 9-cis-Icosenoyl bedeuten,

c) eine Verbindung oder eine Kombination von Verbindungen aus der Gruppe der Antiphlogistika und/oder Antiinflammatorika, Antibiotika, Antineoplastika und Immunmodulatoren und eine pharmazeutisch annehmbare Trägerflüssigkeit, welche auf pH 7,2—7,4 abgepuffert ist.

6. Verfahren zur Herstellung von pharmazeutischen Zusammensetzungen gemäss Anspruch 1 in Form einer Liposomendispersion enthaltend

a) Natrium- oder Dinatrium-N-[1,2-di-(9-cis-octadecenoyl)-sn-glycero-3-phosphoethanol]-N-hydroxysuccinylamin oder Natrium- oder Dinatrium-N-[1,2-di-(9-cis-octadecenoyl)-sn-glycero-3-phosphoethanol]-N-hydroxyglutarylamin,

b) 1,2-Di-(9-cis-octadecenoyl)-sn-glycero-3-phosphoethanolamin,

c) eine Verbindung oder eine Kombination von Verbindungen aus der Gruppe der Antiphlogistika und/oder Antiinflammatorika, Antibiotika, Antineoplastika und Immunmodulatoren, und eine pharmazeutisch annehmbare Trägerflüssigkeit, welche auf pH 7,2—7,4 abgepuffert ist.

7. Verfahren zur Herstellung von pharmazeutischen Zusammensetzungen gemäss Anspruch 1 in Form einer Liposomendispersion enthaltend

a) Natrium- oder Dinatrium-N-[1,2-di-(9-cis-octadecenoyl)-sn-glycero-3-phosphoethanol]-N-hydroxysuccinylamin oder Natrium- oder Dinatrium-N-[1,2-di-(9-cis-octadecenoyl)-sn-glycero-3-phosphoethanol]-N-hydroxyglutarylamin,

b) 1,2-Di-(9-cis-octadecenoyl)-sn-glycero-3-phosphoethanolamin,

c) eine Verbindung oder Kombination von Verbindungen aus der Gruppe Diclofenac, Pirprofen, Mitomycin, Cytarabin, Dactinomycin, Daunorubicin, Doxorubicin, Etoposid, N - Acetyl - D - muramyl - L - alanyl - D - isoglutaminyl - L - alanin - 2 - (1,2 - dipalmitoyl - sn - glycero - 3 - hydroxyphosphoryloxy) - ethylamid, N - Acetyl - D - muramyl - L - alanyl - D - glutaminsäure - ($C^{\gamma}$ - L - alanin - 2 - (1,2 - dipalmitoyl - sn - glycero - 3 - hydroxyphosphoryloxy) - ethylamid) - dinatriumsalz, N - Acetyl - D - muramyl - L - alanyl - D - isoglutamin - natriumsalz, N - Acetyldesmethyl - muramyl - L - alanyl - D - isoglutamin-natriumsalz, N - Acetyl - D - muramyl - L - alanyl - D - glutamin - α - n - butylester, $N^{\alpha}$ - (N - acetylmuramyl - L - alanyl - D - isoglutaminyl) - $N^{\gamma}$ - stearoyl - L - lysin, 6 - O - stearoyl - N - acetyl - D - muramyl - L - alanin - D - isoglutamin und Lymphokin, und gegebenenfalls eine pharmazeutisch annehmbare Trägerflüssigkeit, welche auf pH 7,2—7,4 abgepuffert ist.

**Revendications pour les Etats contractants: BE CH DE FR GB IT LI LU NL SE**

1. Compositions pharmaceutiques à l'état de dispersions de liposomes consistant en

a) un phospholipide de formule

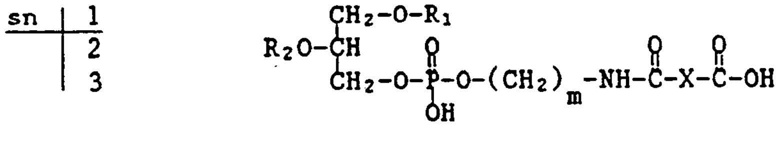

(I),

dans laquelle m est égal à 2 ou 3, $R_1$ et $R_2$ représentent chacun, indépendamment l'un de l'autre, un groupe alkyle, alcényle ou acyle, contenant chacun 10 à 20 atomes de carbone et X représente une liaison directe, un groupe alkylène en C1—C4, alcénylène en C2—C4 ou un groupe alkylène en C1—C4 substitué par des groupes hydroxy, ou un sel acceptable pour l'usage pharmaceutique d'un tel phospholipide.

b) un phospholipide de formule

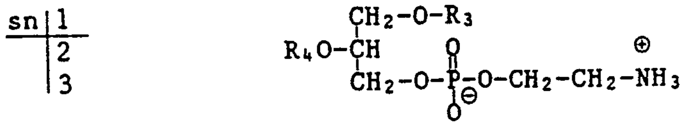

(II),

dans laquelle $R_3$ et $R_4$ représentent chacun le radical acyle d'un acide carboxylique saturé ou insaturé contenant 10 à 20 atomes de carbone et une à deux doubles liaisons,

c) un composé ou un mélange de composés possédant des propriétés pharmacologiques, et le cas échéant

d) un lipide du groupe formé par la phosphatidylcholine, la phosphatidylsérine, le phosphatidylinositol, le phosphatidylglycérol, la cardiolipine et le cholestérol et, le cas échéant, une solution véhicule acceptable pour l'usage pharmaceutique, tamponnée à pH 7,0—7,8, et le cas échéant des produits auxiliaires acceptables pour l'usage pharmaceutique.

2. Compositions pharmaceutiques selon la revendication 1, contenant

a) un phospholipide de formule I dans laquelle m est égal à 2, $R_1$ et $R_2$ ont les significations indiquées ci-dessus et X représente un groupe alkylène en C1—C4, alcénylène en C2—C4 ou un groupe alcénylène en C2—C4 substitué par des groupes hydroxy, ou un sel acceptable pour l'usage pharmaceutique d'un tel phospholipide,

b) un phospholipide de formule II dans laquelle $R_3$ et $R_4$ représentent chacun, indépendamment l'un de l'autre, un groupe alcényoyle à chaîne droite en C10—C20,

c) un composé ou une combinaison de composés possédant des propriétés pharmacologiques, et le cas échéant un liquide véhicule acceptable pour l'usage pharmaceutique, tamponné à pH 7,2—7,4.

3. Compositions pharmaceutiques selon la revendication 1, contenant

a) un phospholipide de formule I dans laquelle m est égal à 2, $R_1$ et $R_2$ ont les significations indiquées ci-dessus et X représente un groupe alkylène en C2—C4 ou alcénylène en C2—C4, ou un sel acceptable pour l'usage pharmaceutique d'un tel phospholipide,

b) un phospholipide de formule II dans laquelle $R_3$ et $R_4$ représentent chacun, indépendamment l'un de l'autre, un groupe alcanoyle à chaîne droite en C10—C20,

c) un composé ou une combinaison de composés pris dans le groupe formé par les antiphlogistiques et/ou les anti-inflammatoires, les antiobiotiques, les agents antileishmania, les antinéoplastiques et les immunomodulateurs et le cas échéant un liquide véhicule acceptable pour l'usage pharmaceutique, tamponné à pH 7,2—7,4.

4. Compositions pharmaceutiques selon la revendication 1, à l'état de dispersions de liposomes contenant

a) un phospholipide de formule I dans laquelle m est égal à 2, $R_1$ et $R_2$ représentent chacun, indépendamment l'un de l'autre, un groupe alcanoyle ou alcénoyle à chaîne droite et à nombre pair d'atomes de carbone, de 10 à 20, et X représente un groupe alkylène en C2—C4 ou alcénylène en C2—C4, ou un sel acceptable pour l'usage pharmaceutique d'un tel phospholipide,

b) un phospholipide de formule II dans laquelle $R_3$ et $R_4$ représentent des groupes alcénoyle à chaîne droite en C10—C20 et à nombre pair d'atomes de carbone, de 10 à 20,

c) un composé ou une combinaison de composés possédant des propriétés pharmacologiques et pris dans le groupe formé par les antiphlogistiques et/ou les anti-inflammatoires, les antibiotiques, les agents antileishmania, les antinéoplastiques et les immunomodulateurs, et un liquide véhicule acceptable pour l'usage pharmaceutique, tamponné à pH 7,2—7,4.

5. Compositions pharmaceutiques selon la revendication 1, à l'état de dispersions de liposomes contenant

a) un phospholipide de formule I dans laquelle m est égal à 2, $R_1$ et $R_2$ représentent chacun, indépendamment l'un de l'autre, un groupe 9-cis-dodécénoyle, 9-cis-tétradécénoyle, 9-cis-hexadécénoyle, 6-cis-, 6-trans-, 9-cis-, 9-trans- ou 11-cis-octadécénoyle ou 9-cis-éicosénoyle, et X représente un groupe alkylène en C2—C4 ou alcénylène en C2—C4, ou un sel acceptable pour l'usage pharmaceutique d'un tel phospholipide,

b) un phospholipide de formule II dans laquelle $R_3$ et $R_4$ représentent chacun, indépendamment l'un de l'autre, un groupe 9-cis-dodécénoyle, 9-cis-tétradécénoyle, 9-cis-hexadécénoyle, 6-cis-, 6-trans-, 9-cis-, 9-trans ou 11-cis-octadécénoyle ou 9-cis-éicosénoyle,

c) un composé ou une combinaison de composés pris dans le groupe formé par les antiphlogistiques et/ou les anti-inflammatoires, les antibiotiques, les antinéoplastiques et les immunomodulateurs, et un liquide véhicule acceptable pour l'usage pharmaceutique, tamponné à pH 7,2—7,4.

6. Compositions pharmaceutiques selon la revendication 1, à l'état de dispersions de liposomes contenant

a) le sel monosodique ou disodique de la N-[1,2-di-(9-cis-octadécénoyl)-sn-glycéro-3-phosphoéthanol]-N-hydroxysuccinylamine ou le sel monosodique ou disodique de la N-[1,2-di-(9-cis-octadécénoyl)-sn-glycéro-3-phosphoéthanol]-N-hydroxyglutarylamine,

b) la 1,2-di-(9-cis-octadécénoyl)-sn-glycéro-3-phosphoéthanolamine,

c) un composé ou une combinaison de composés pris dans le groupe formé par les antiphlogistiques et/ou les anti-inflammatoires, les antibiotiques, les antinéoplastiques et les immunomodulateurs, et un liquide véhicule acceptable pour l'usage pharmaceutique, tamponné à pH 7,2—7,4.

7. Compositions pharmaceutiques selon la revendication 1, à l'état de dispersions de liposomes contenant

a) le sel monosodique ou disodique de la N-[1,2-di-(9-cis-octadécénoyl)-sn-glycéro-3-phosphoéthanol]-N-hydroxysuccinylamine ou le sel monosodique ou disodique de la N-[1,2-di-(9-cis-octadécénoyl)-sn-glycéro-3-phosphoéthanol]-N-hydroxyglutarylamine,

b) la 1,2-di-(9-cis-octadécénoyl)-sn-glycéro-3-phosphoéthanolamine,

c) un composé ou une combinaison de composés pris dans le groupe formé par le Diclofenac, le Pirprofen, la Mitomycine, la Cytarabine, la Dactinomycine, la Daunorubicine, Doxorubicine, l'Etoposid, le 2 - [1,2 - dipalmitoyl - sn - glycéro - 3 - hydroxy - phosphoryloxy) - éthylamide de la N - acétyl - D - muramyl - L - alanyl - D - isoglutaminyl - L - alanine, le sel disodique du [$C^{gamma}$ - L - alanine - 2 - (1,2 - dipalmitoyl - sn - glycéro - 3 - hydroxy - phosphoryloxy) - ethylamide de l'acide N - acétyl - D - muramyl - L - alanyl - D - glutamique), le sel de sodium de la N - acétyl - D - muramyl - L - alanyl - D - isoglutamine, le sel de sodium de la N - acétyldesméthyl - muramyl - L - alanyl - D - isoglutamine, le N - acétyl - D - muramyl - L - alanyl - D - glutamate d'alpha - n - butyle, la $N^{alpha}$ - (N - acétyl - D - muramyl - L - alanyl - D - isoglutaminyl) - $N^{gamma}$ - stéaroyl - L - lysine, la 6 - O - stéaroyl - N - acétyl - D - muramyl - L - alanine - D - isoglutamine et une lymphokine, et un liquide véhicule acceptable pour l'usage pharmaceutique, tamponné à pH 7,2—7,4.

8. Mélanges convenant à la préparation de liposomes consistant en phospholipides de formules I et II et un composé ou mélange de composés possédant des propriétés pharmacologiques selon la revendication 1.

9. Procédé de préparation des compositions pharmaceutiques selon l'une des revendications 1 à 7, caractérisé en ce que

a) on prépare un mélange homogène consistant en les phospholipides de formules I et II et les lipides supplémentaires mentionnés et un composé ou mélange de composés lipophiles possédant des propriétés pharmacologiques, et pour le préparation des liposomes, on disperse le mélange homogène ainsi obtenu en phase aqueuse, ou bien

b) on prépare un mélange homogène consistant en les phospholipides de formules I et II et les lipides supplémentaires mentionnées et pour la préparation des liposomes, on disperse le mélange homogène ainsi obtenu dans une phase aqueuse contenant un composé ou mélange de composés solubles dans l'eau et possédant des propriétés pharmacologiques et,

lorsque c'est nécessaire, on tamponne la dispersion aqueuse ainsi obtenue à pH 7,0—7,8 et si on le désire, on enrichit et/ou on sépare les liposomes ainsi obtenus.

10. Systèmes d'administration selon la revendication 1, pour l'utilisation pour le traitement du corps humain ou animal.

**Revendications pour l'Etats contractant: AT**

1. Procédé de préparation de compositions pharmaceutiques à l'état de dispersions de liposomes consistant en

a) un phospholipide de formule

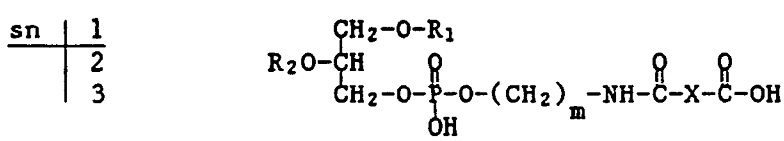

(I),

dans laquelle m est égal à 2 ou 3, $R_1$ et $R_2$ représentent chacun, indépendamment l'un de l'autre, un groupe alkyle, alcényle ou acyle contenant chacun 10 à 20 atomes de carbone et X représente une liaison directe, un groupe alkylène en C1—C4, alcénylène en C2—C4, ou un groupe alkylène en C1—C4 ou alcényle en C2—C4 substitué par des groupes hydroxy, ou un sel acceptable pour l'usage pharmaceutique d'un tel phospholipide,

b) un phospholipide de formule

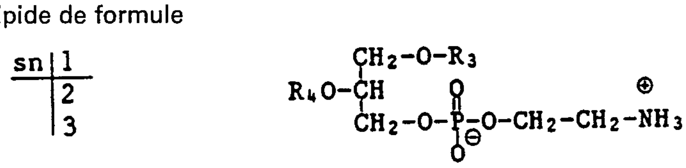

(II),

dans laquelle $R_3$ et $R_4$ représentent chacun les radicaux acyle d'acides carboxyliques saturés ou insaturés contenant 10 à 20 atomes de carbone et une à deux doubles liaisons,

c) un composé ou un mélange de composés possédant des propriétés pharmacologiques et le cas échéant,

d) un lipide du groupe formé par la phosphatidylcholine, la phosphatidylsérine, le phosphatidylinositol, le phosphatidylglycérol, la cardiolipine et le cholestérol, et une solution véhicule acceptable pour l'usage pharmaceutique, tamponnée à pH 7,0—7,8, et le cas échéant des produits auxiliaires acceptables pour l'usage pharmaceutique caractérisé en ce que

a) on prépare un mélange homogène consistant en les phospholipides de formules I et II et les lipides supplémentaires mentionnés et un composé ou mélange de composés lipophiles possédant des propriétés pharmacologiques, et pour le préparation des liposomes, on disperse le mélange homogène ainsi obtenu en phase aqueuse, ou bien

b) on prépare un mélange homogéne consistant en les phospholipides de formules I et II et les lipides supplémentaires mentionnées, et pour la préparation des liposomes, on disperse le mélange homogène ainsi obtenu dans une phase aqueuse contenant un composé ou mélange de composés solubles dans l'eau et possédant des propriétés pharmacologiques et,

lorsque c'est nécessaire, on tamponne la dispersion aqueuse ainsi obtenue à pH 7,0—7,8 et, si on le désire, on enrichit les liposomes ainsi obtenus et/ou on les sépare.

2. Procédé de préparation de compositions pharmaceutiques selon la revendication 1, à l'état de dispersions de liposomes contentant

a) un phospholipide de formule I dans laquelle m est égal à 2, $R_1$ et $R_2$ ont les significations indiquées ci-dessus et X représente un groupe alkylène en C1—C4, alcénylène en C2—C4 ou un groupe alkylène en C1—C4 substitué par des groupes hydroxy, ou un sel acceptable pour l'usage pharmaceutique d'un tel phospholipide,

b) un phospholipide de formule II dans laquelle $R_3$ et $R_4$ représentent chacun, indépendamment l'un de l'autre, un groupe alcénoyle à chaîne droite en C10—C20,

c) un composé ou une combinaison de composés possédant des propriétés pharmacologiques et un liquide véhicule acceptable pour l'usage pharmaceutique, tamponné à pH 7,2—7,4.

3. Procédé de préparation de compositions pharmaceutiques selon la revendication 1 à l'état de dispersions de liposomes contenant

a) un phospholipide de formule I dans laquelle m est égal à 2, $R_1$ et $R_2$ ont les significations indiquées ci-dessus et X représente un groupe alkylène en C2—C4 ou alcénylène en C2—C4, ou un sel acceptable pour l'usage pharmaceutique d'un tel phospholipide,

b) un phospholipide de formule II dans laquelle $R_3$ et $R_4$ représentent chacun, indépendamment l'un de l'autre, un groupe alcénoyle à chaîne droite en C10—C20,

c) un composé ou une combinaison de composés pris dans le groupe formé par les antiphlogistiques et/ou les anti-inflammatoires, les antibiotiques, les agents antileishmania, les antinéoplastiques et les immunomodulateurs, et un liquide véhicule acceptable pour l'usage pharmaceutique, tamponné à pH 7,2—7,4.

4. Procédé de préparation de compositions pharmaceutiques selon la revendication 1, à l'état de dispersions de liposomes contenant

a) un phospholipide de formule I dans laquelle m est égal à 2, $R_1$ et $R_2$ représentent chacun, indépendamment l'un de l'autre, un groupe alcanoyle ou alcénoyle à chaîne droite et nombre pair d'atomes de carbone, de 10 à 20, et X représente un groupe alkylène en C2—C4 ou alcénylène en C2—C4, ou un sel acceptable pour l'usage pharmaceutique d'un tel phospholipide,

b) un phospholipide de formule II dans laquelle $R_3$ et $R_4$ représentent chacun un groupe alcénoyle à chaîne droite en C10—C20 et nombre pair d'atomes de carbone, de 10 à 20,

c) un composé ou une combinaison de composés possédant des propriétés pharmacologiques et pris dans le groupe formé par les antiphlogistiques et/ou les anti-inflammatoires, les antibiotiques, les agents antileishmania, les antinéoplastiques et les immunomodulateurs, et un liquide véhicule acceptable pour l'usage pharmaceutique, tamponné à pH 7,2—7,4.

5. Procédé de préparation de compositions pharmaceutiques selon la revendication 1 à l'état de dispersions de liposomes contenant

a) un phospholipide de formule I dans laquelle m est égal à 2, $R_1$ et $R_2$ représentent chacun, indépendamment l'un de l'autre, un groupe 9-cis-dodécénoyle, 9-cis-tétradécénoyle, 9-cis-hexadécénoyle, 6-cis-, 6-trans-, 9-cis-, 9-trans- ou 11-cis-octadécénoyle ou 9-cis-éicosénoyle, et X représente un groupe alkylène en C2—C4 ou alcénylène en C2—C4, ou un sel acceptable pour l'usage pharmaceutique d'un tel phospholipide,

b) un phospholipide de formule II dans laquelle $R_3$ et $R_4$ représentent chacun, indépendamment l'un de l'autre, un groupe 9-cis-dodécénoyle, 9-cis-tétradécénoyle, 9-cis-hexadécénoyle, 6-cis-, 6-trans-, 9-cis-, 9-trans ou 11-cis-octadécénoyle ou 9-cis-éicosénoyle,

c) un composé ou une combinaison de composés pris dans le groupe formé par les antiphlogistiques et/ou les anti-inflammatoires, les antibiotiques, les antinéoplastiques et les immunomodulateurs, et un liquide véhicule acceptable pour l'usage pharmaceutique, tamponné à pH 7,2—7,4.

6. Procédé de préparation de compositions pharmaceutiques selon la revendication 1 à l'état de dispersions de liposomes contenant

a) le sel monosodique ou disodique de la N-[1,2-di-(9-cis-octadécénoyl)-sn-glycéro-3-phosphoéthanol]-N-hydroxysuccinylamine ou le sel monosodique ou disodique de la N-[1,2-di-(9-cis-octadécénoyl)-sn-glycéro-3-phosphoéthanol]-N-hydroxyglutarylamine,

b) la 1,2-di-(9-cis-octadécénoyl)-sn-glycéro-3-phosphoéthanolamine,

c) un composé ou une combinaison de composés pris dans le groupe formé par les antiphlogistiques et/ou les anti-inflammatoires, les antibiotiques, les antinéoplastiques et les immunomodulateurs, et un liquide véhicule acceptable pour l'usage pharmaceutique, tamponné à pH 7,2—7,4.

7. Procédé de préparation de compositions pharmaceutiques selon la revendication 1 à l'état de dispersions de liposomes contenant

a) le sel monosodique ou disodique de la N-[1,2-di-(9-cis-octadécénoyl)-sn-glycéro-3-phosphoéthanol]-N-hydroxysuccinylamine ou le sel monosodique ou disodique de la N-[1,2-di-(9-cis-octadécénoyl)-sn-glycéro-3-phosphoéthanol]-N-hydroxyglutarylamine,

b) la 1,2-di-(9-cis-octadécénoyl)-sn-glycéro-3-phosphoéthanolamine,

c) un composé ou une combinaison de composés pris dans le groupe formé par le Diclofenac, le Pirprofen, la Mitomycine, la Cytarabine, la Dactinomycine, la Daunorubicine, Doxorubicine, l'Etoposid, le 2 - [1,2 - dipalmitoyl - sn - glycéro - 3 - hydroxy-phosphoryloxy) - éthylamide de la N - acétyl - D - muramyl - L - alanine - D - isoglutaminyl - L - alanine, le sel disodique du $C^{gamma}$ - L - alanine - 2 - (1,2 - dipalmitoyl - sn - glycéro - 3 - hydroxy-phosphoryloxy) - ethylamide de l'acide N - acétyl - D - muramyl - L - alanyl - D - glutamique, le sel de sodium de la N - acétyl - D - muramyl - L - alanyl - D - isoglutamine, le sel de sodium de la N - acétyldesméthyl - muramyl - L - alanyl - D - isoglutamine, l'ester alpha-n-butylique de la N - acétyl - D - muramyl - L - alanyl - D - glutamine, la $N^{alpha}$ - (L - acétyl-muramyl - L - alanyl - D - isoglutaminyl) - $N^{gamma}$ - stéaroyl - L - lysine, la 6 - O - stéaroyl - N - acétyl - D - muramyl - L - alanine - D - isoglutamine et une lymphokine, et le cas échéant un liquide véhicule acceptable pour l'usage pharmaceutique, tamponné à pH 7,2—7,4.

**Claims for the Contracting States: BE CH DE FR GB IT LI LU NL SE**

1. Pharmaceutical compositions in the form of a liposome dispersion consisting of

a) a phospholipid of the formula

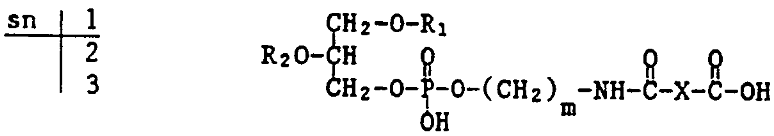
(I),

wherein m represents two or three, $R_1$ and $R_2$ independently of each other represent alkyl, alkenyl or acyl each having from 10 to 20 carbon atoms, and X represents a direct bond, $C_1$—$C_4$alkylene, $C_2$—$C_4$alkenylene or $C_1$—$C_4$alkylene substituted by hydroxy, or a pharmaceutically acceptable salt thereof,

b) a phospholipid of the formula

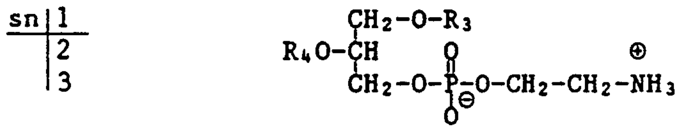
(II),

wherein $R_3$ and $R_4$ represent the acyl group of a saturated or unsaturated carboxylic acid having from 10 to 20 carbon atoms and 1 or 2 double bonds,

c) a compound or a mixture of compounds having pharmacological properties and, optionally,

d) a lipid from the group consisting of phosphatidylcholine, phosphatidylserine, phosphatidylinositol, phosphatidylglycerol, cardiolipin and cholesterol and, optionally, a pharmaceutically acceptable carrier solution buffered to pH 7.0—7.8 and, optionally, pharmaceutically acceptable adjuncts.

2. Pharmaceutical compositions according to claim 1, containing

a) a phospholipid of the formula I, wherein m represents two, $R_1$ and $R_2$ are as defined hereinbefore and X represents $C_1$—$C_4$alkylene, $C_2$—$C_4$alkenylene or $C_2$—$C_4$alkenylene substituted by hydroxy, or a pharmaceutically acceptable salt thereof,

b) a phospholipid of the formula II, wherein $R_3$ and $R_4$ independently of each other represent straight-chained $C_{10}$—$C_{20}$alkenoyl,

c) a compound or a combination of compounds having pharmacological properties and, optionally, a pharmaceutically acceptable carrier liquid buffered to pH 7.2—7.4.

3. Pharmaceutical compositions according to claim 1 containing

a) a phospholipid of the formula I, wherein m represents two, $R_1$ and $R_2$ are as defined hereinbefore and X represents $C_2$—$C_4$alkylene or $C_2$—$C_4$alkenylene, or a pharmaceutically acceptable salt thereof,

b) a phospholipid of the formula II, wherein $R_3$ and $R_4$ independently of each other represent straight-chained $C_{10}$—$C_{20}$alkanoyl,

c) a compound or a combination of compounds from the group consisting of antiphlogistics and/or antiinflammatory agents, antibiotics, antileishmaniasis compounds, antineoplastic agents and, optionally, a pharmaceutically acceptable carrier liquid buffered to pH 7.2—7.4.

4. Pharmaceutical compositions according to claim 1 in the form of a liposome dispersion containing

a) a phospholipid of the formula I, wherein m represents two, $R_1$ and $R_2$ independently of each other represent straight-chained alkanoyl or alkenoyl having an even number of from 10 to 20 carbon atoms, and X represents $C_2$—$C_4$alkylene or $C_2$—$C_4$alkenylene, or a pharmaceutically acceptable salt thereof,

b) a phospholipid of the formula II, wherein $R_3$ and $R_4$ represent straight-chained $C_{10}$—$C_{20}$alkenoyl having an even number of from 10 to 20 carbon atoms,

c) a compound or a combination of compounds having pharmacological properties from the group consisting of antiphlogistics and/or antiinflammatory agents, antibiotics, antileishmaniasis compounds, antineoplastic agents and immunomodulators, and a pharmaceutically acceptable carrier liquid buffered to pH 7.2—7.4.

5. Pharmaceutical compositions according to claim 1 in the form of a liposome dispersion containing

a) a phospholipid of the formula I, wherein m represents two, $R_1$ and $R_2$ independently of each other represent 9-cis-dodecenoyl, 9-cis-tetradecenoyl, 9-cis-hexadecenoyl, 6-cis-, 6-trans-, 9-cis-, 9-trans- or 11-cis-octadecenoyl, or 9-cis-icosenoyl, and X represents $C_2$—$C_4$alkylene or $C_2$—$C_4$alkenylene, or a pharmaceutically acceptable salt thereof.

b) a phospholipid of the formula II, wherein $R_3$ and $R_4$ independently of each other represent 9-cis-dodecenoyl, 9-cis-tetradecenoyl, 9-cis-hexadecenoyl, 6-cis-, 6-trans-, 9-cis-, 9-trans or 11-cis-octadecenoyl, or 9-cis-icosenoyl,

c) a compound or a combination of compounds from the group consisting of antiphlogistics and/or antiinflammatory agents, antibiotics, antineoplastic agents and immunomodulators, and a pharmaceutically acceptable carrier liquid buffered to pH 7.2—7.4.

6. Pharmaceutical compositions according to claim 1 in the form of a liposome dispersion containing

a) sodium- or disodium-N-[1,2-di-(9-cis-octadecenoyl)-sn-glycero-3-phosphoethanol]-N-hydroxysuccinylamine or sodium- or disodium-N-[1,2-di-(9-cis-octadecenoyl)-sn-glycero-3-phosphoethanol]-N-hydroxyglutarylamine,

b) 1,2-di-(9-cis-octadecenoyl)-sn-glycero-3-phosphoethanolamine,

c) a compound or a combination of compounds from the group consisting of antiphlogistics and/or antiinflammatory agents, antibiotics, antineoplastic agents and immunomodulators, and a pharmaceutically acceptable carrier liquid buffered to pH 7.2—7.4.

7. Pharmaceutical compositions according to claim 1 in the form of a liposome dispersion containing

a) sodium- or disodium-N-[1,2-di-(9-cis-octadecenoyl)-sn-glycero-3-phosphoethanol]-N-hydroxysuccinylamine or sodium- or disodium-N-[1,2-di-(9-cis-octadecenoyl)-sn-glycero-3-phosphoethanol]-N-hydroxyglutarylamine,

b) 1,2-di-(9-cis-octadecenoyl)-sn-glycero-3-phosphoethanolamine,

c) a compound or combination of compounds from the group consisting of diclofenac, pirprofen, mitomycin, cytarabine, dactinomycin, daunorubicine, doxorubicine, etoposide, N - acetyl - D - muramyl - L - alanyl - D - isoglutaminyl - L - alanine - 2 - (1,2 - dipalmitoyl - sn - glycero - 3 - hydroxyphosphoryloxy) - ethylamide, N - acetyl - D - muramyl - L - alanyl - D - glutamic acid - ($C^{\gamma}$ - L - alanine - 2 - (1,2 - dipalmitoyl - sn - glycero - 3 - hydroxyphosphoryloxy) - ethylamide disodium salt, N - acetyl - D - muramyl - L - alanyl - D - isoglutamine sodium salt, N - acetyldemethyl - muramyl - L - alanyl - D - isoglutamine sodium salt, N - acetyl - D - muramyl - L - alanyl - D - glutamine - α - n - butyl ester, $N^{\alpha}$ - (N - acetyl - D - muramyl - L - alanyl - D - isoglutaminyl) - $N^{\gamma}$ - stearoyl - L - lysine, 6 - O - stearoyl - N - acetyl - D - muramyl - L - alanine - D - isoglutamine and lymphokine, and a pharmaceutically acceptable carrier liquid buffered to pH 7.2—7.4.

8. Mixtures suitable for the manufacture of liposomes consisting of phospholipids of formulae I and II and a compound or mixture of compounds having pharmacological properties according to claim 1.

9. A process for the manufacture of pharmaceutical compositions according to any one of claims 1 to 7, characterised in that

a) a homogeneous mixture consisting of the phospholipids of formulae I and II and the mentioned additional lipids and a lipophilic compound or mixture of compounds having pharmacological properties is prepared and, in order to produce liposomes, the obtainable homogeneous mixture is dispersed in an aqueous phase, or

b) a homogeneous mixture consisting of the phospholipids of formula I and II and the mentioned additional lipids is prepared and, in order to produce liposomes, the obtainable homogeneous mixture is dispersed in an aqueous phase containing a water-soluble compound or mixture of compounds having pharmacological properties, and

if necessary, the obtainable aqueous dispersion is buffered to pH 7.0—7.8 and, if desired, the obtainable liposomes are concentrated and/or separated.

10. Administration systems according to claim 1 for use in the treatment of the human or animal body.

**Claims for the Contracting State: AT**

1. A process for the production of pharmaceutical compositions in the form of a liposome dispersion consisting of

a) a phospholipid of the formula

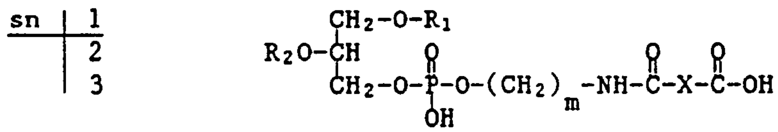 (I),

wherein m represents two or three, $R_1$ and $R_2$ independently of each other represent alkyl, alkenyl or acyl each having from 10 to 20 carbon atoms, and X represents a direct bond, $C_1$—$C_4$alkylene, $C_2$—$C_4$alkenylene or $C_1$—$C_4$alkylene or $C_2$—$C_4$alkenyl substituted by hydroxy, or a pharmaceutically acceptable salt thereof,

b) a phospholipid of the formula

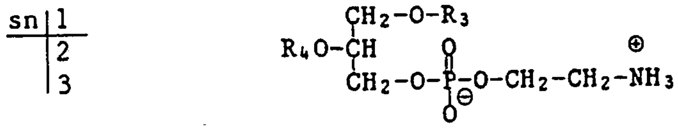 (II),

wherein $R_3$ and $R_4$ represent the acyl group of a saturated or unsaturated carboxylic acid having from 10 to 20 carbon atoms and 1 or 2 double bonds,

c) a compound or a mixture of compounds having pharmacological properties and, optionally,

d) a lipid from the group consisting of phosphatidylcholine, phosphatidylserine, phosphatidylinositol, phosphatidylglycerol, cardiolipin and cholesterol and a pharmaceutically acceptable carrier solution buffered to pH 7.0—7.8 and, optionally, pharmaceutically acceptable adjuncts, characterised in that

a) a homogeneous mixture consisting of the phospholipids of formulae I and II and the mentioned additional lipids and a lipophilic compound or mixture of compounds having pharmacological properties is prepared and, in order to produce liposomes, the obtainable homogeneous mixture is dispersed in an aqueous phase, or

b) a homogeneous mixture consisting of the phospholipids of formulae I and II and the mentioned additional lipids is prepared and, in order to produce liposomes, the obtainable homogeneous mixture is dispersed in an aqueous phase containing a water-soluble compound or mixture of compounds having pharmacological properties, and

if necessary, the obtainable aqueous dispersion is buffered to pH 7.0—7.8 and, if desired, the obtainable liposomes are concentrated and/or separated.

2. A process for the production of pharmaceutical compositions according to claim 1 in the form of a liposome dispersion containing

a) a phospholipid of the formula I, wherein m represents two, $R_1$ and $R_2$ are as defined hereinbefore and X represents $C_1$—$C_4$alkylene, $C_2$—$C_4$alkenylene or $C_1$—$C_4$alkylene substituted by hydroxy, or a pharmaceutically acceptable salt thereof,

b) a phospholipid of the formula II, wherein $R_3$ and $R_4$ independently of each other represent straight-chained $C_{10}$—$C_{20}$alkenoyl,

c) a compound or a combination of compounds having pharmacological properties and a pharmaceutically acceptable carrier liquid buffered to pH 7.2—7.4.

3. A process for the production of pharmaceutical compositions according to claim 1 in the form of a liposome dispersion containing

a) a phospholipid of the formula I, wherein m represents two, $R_1$ and $R_2$ are as defined hereinbefore and X represents $C_2$—$C_4$alkylene or $C_2$—$C_4$alkenylene, or a pharmaceutically acceptable salt thereof,

b) a phospholipid of the formula II, wherein $R_3$ and $R_4$ independently of each other represent straight-chained $C_{10}$—$C_{20}$alkanoyl,

c) a compound or a combination of compounds from the group consisting of antiphlogistics and/or antiinflammatory agents, antibiotics, antileishmaniasis compounds, antineoplastic agents and immunomodulators, and a pharmaceutically acceptable carrier liquid buffered to pH 7.2—7.4.

4. A process for the production of pharmaceutical compositions according to claim 1 in the form of a liposome dispersion containing

a) a phospholipid of the formula I, wherein m represents two, $R_1$ and $R_2$ independently of each other represent straight-chained alkanoyl or alkenoyl having an even number of from 10 to 20 carbon atoms, and X represents $C_2$—$C_4$alkylene or $C_2$—$C_4$alkenylene, or a pharmaceutically acceptable salt thereof,

b) a phospholipid of the formula II, wherein $R_3$ and $R_4$ represent straight-chained $C_{10}$—$C_{20}$alkenoyl having an even number of from 10 to 20 carbon atoms,

c) a compound or a combination of compounds having pharmacological properties from the group consisting of antiphlogistics and/or antiinflammatory agents, antibiotics, antileishmaniasis compounds, antineoplastic agents and immunomodulators, and a pharmaceutically acceptable carrier liquid buffered to pH 7.2—7.4.

5. A process for the production of pharmaceutical compositions according to claim 1 in the form of a liposome dispersion containing

a) a phospholipid of the formula I, wherein m represents two, $R_1$ and $R_2$ independently of each other represent 9-cis-dodecenoyl, 9-cis-tetradecenoyl, 9-cis-hexadecenoyl, 6-cis-, 6-trans-, 9-cis-, 9-trans- or 11-

cis-octadecenoyl, or 9-cis-icosenoyl, and X represents $C_2$—$C_4$alkylene or $C_2$—$C_4$alkenylene, or a pharmaceutically acceptable salt thereof,

b) a phospholipid of the formula II, wherein $R_3$ and $R_4$ independently of each other represent 9-cis-dodecenoyl, 9-cis-tetradecenoyl, 9-cis-hexadecenoyl, 6-cis-, 6-trans-, 9-cis-, 9-trans or 11-cis-octadecenoyl, or 9-cis-icosenoyl,

c) a compound or a combination of compounds from the group consisting of antiphlogistics and/or antiinflammatory agents, antibiotics, antineoplastic agents and immunomodulators, and a pharmaceutically acceptable carrier liquid buffered to pH 7.2—7.4.

6. A process for the production of pharmaceutical compositions according to claim 1 in the form of a liposome dispersion containing

a) sodium- or disodium-N-[1,2-di-(9-cis-octadecenoyl)-sn-glycero-3-phosphoethanol]-N-hydroxy-succinylamine or sodium- or disodium-N-[1,2-di-(9-cis-octadecenoyl)-sn-glycero-3-phosphoethanol]-N-hydroxyglutarylamine,

b) 1,2-di-(9-cis-octadecenoyl)-sn-glycero-3-phosphoethanolamine,

c) a compound or a combination of compounds from the group consisting of antiphlogistics and/or antiinflammatory agents, antibiotics, antineoplastic agents and immunomodulators, and a pharmaceutically acceptable carrier liquid buffered to pH 7.2—7.4.

7. A process for the production of pharmaceutical compositions according to claim 1 in the form of a liposome dispersion containing

a) sodium- or disodium-N-[1,2-di-(9-cis-octadecenoyl)-sn-glycero-3-phosphoethanol]-N-hydroxy-succinylamine or sodium- or disodium-N-[1,2-di-(9-cis-octadecenoyl)-sn-glycero-3-phosphoethanol]-N-hydroxyglutarylamine,

b) 1,2-di-(9-cis-octadecenoyl)-sn-glycero-3-phosphoethanolamine,

c) a compound or combination of compounds from the group consisting of diclofenac, pirprofen, mitomycin, cytarabine, dactinomycin, daunorubicine, doxorubicine, etoposide, N - acetyl - D - muramyl - L - alanyl - D - isoglutaminyl - L - alanine - 2 - (1,2 - dipalmitoyl - sn - glycero - 3 - hydroxy-phosphoryloxy) - ethylamide, N - acetyl - D - muramyl - L - alanyl - D - glutamic acid - ($C^{\gamma}$ - L - alanine - 2 - (1,2 - dipalmitoyl - sn - glycero - 3 - hydroxyphosphoryloxy) - ethylamide disodium salt, N - acetyl - D - muramyl - L - alanyl - D - isoglutamine sodium salt, N - acetyldemethyl - muramyl - L - alanyl - D - isoglutamine sodium salt, N - acetyl - D - muramyl - L - alanyl - D - glutamine - α - n - butyl ester, $N^{\alpha}$ - (N - acetylmuramyl - L - alanyl - D - isoglutaminyl) - $N^{\gamma}$ - stearoyl - L - lysine, 6 - O - stearoyl - N - acetyl - D - muramyl - L - alanine - D - isoglutamine and lymphokine, and optionally a pharmaceutically acceptable carrier liquid buffered to pH 7.2—7.4.